(19) Europäisches Patentamt
European Patent Office
Office européen des brevets

(11) EP 3 122 847 B1

(12) **EUROPEAN PATENT SPECIFICATION**

(45) Date of publication and mention of the grant of the patent:
**15.05.2019 Bulletin 2019/20**

(21) Application number: **15715933.6**

(22) Date of filing: **26.03.2015**

(51) Int Cl.:
***C11B 9/00*** (2006.01) ***A61Q 13/00*** (2006.01)
***C07C 49/20*** (2006.01) ***C11D 3/50*** (2006.01)
***C07C 255/46*** (2006.01) ***C07C 31/125*** (2006.01)
***C07C 33/025*** (2006.01) ***C07C 33/14*** (2006.01)
***C07D 309/04*** (2006.01) ***C07C 69/013*** (2006.01)
***C07C 69/07*** (2006.01) ***C07C 69/14*** (2006.01)
***C07C 49/563*** (2006.01) ***C07C 255/07*** (2006.01)
***C07C 49/84*** (2006.01) ***C07D 307/00*** (2006.01)
***C07D 307/36*** (2006.01) ***C07D 319/06*** (2006.01)
***C07D 319/08*** (2006.01) ***C07C 233/58*** (2006.01)

(86) International application number:
**PCT/US2015/022616**

(87) International publication number:
**WO 2015/148743 (01.10.2015 Gazette 2015/39)**

(54) **PERFUME SYSTEMS**

DUFTSTOFFSYSTEME

SYSTÈMES DE PARFUM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.03.2014 US 201461970385 P**

(43) Date of publication of application:
**01.02.2017 Bulletin 2017/05**

(60) Divisional application:
**19164800.5**

(73) Proprietor: **The Procter & Gamble Company**
**Cincinnati, OH 45202 (US)**

(72) Inventors:
- **DENUTTE, Hugo, Robert Germain**
  **B-1853 Brussels (BE)**
- **PINTENS, An**
  **B-1853 Brussels (BE)**
- **SMETS, Johan**
  **B-1853 Brussels (BE)**
- **VRIELYNCK, Freek, Annie Camiel**
  **B-8400 Oostende (BE)**
- **VAN AKEN, Koen**
  **B-8400 Oostende (BE)**

(74) Representative: **Yorquez Ramirez, Maria Isabel**
**Procter & Gamble**
**Technical Centres Limited**
**Whitley Road**
**Longbenton**
**Newcastle upon Tyne NE12 9TS (GB)**

(56) References cited:
**WO-A1-2014/089253** **CH-A- 337 832**
**US-A1- 2012 329 696**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 122 847 B1

## Description

FIELD OF INVENTION

The present application relates to perfume raw materials, perfume delivery systems and consumer products comprising such perfume raw materials and/or perfume delivery systems, as well as processes for making and using such perfume raw materials, perfume delivery systems and consumer products.

BACKGROUND OF THE INVENTION

Consumer products may comprise one or more perfumes, and/or perfume delivery systems that can mask an undesirable odor and/or provide a desired scent and/or experience to a product and/or a situs that is contacted with such a product. While current perfumes, and perfume delivery systems provide desirable experiences and/or fragrances, consumers continue to seek products that contain sensates, such as cooling or have scents that may be longer lasting and that are tailored to their individual desires (see for example USPA 2007/0275866 A1 and USPA 2008/0305977 A1) - unfortunately the pool of perfume raw materials and perfume delivery systems that is available is still too limited to completely meet the desired needs.

Applicants believe that the perfume raw materials and perfumes, including the delivery systems, disclosed herein expand the options, as such sensates and/or perfume raw materials can provide variations on character and such and/or perfumes can provide desired sensations and/or odor profiles. In certain aspects, such and/or perfume raw materials and/or perfume delivery systems comprising such and/or perfume raw materials may provide variations on character, sensation and/or odor profiles that are better than expected as measured by parameters such as headspace analysis (employed to determine perfume delivery system perfume leakage and/or perfume delivery efficiency), ClogP, boiling point and/or odor detection threshold.

SUMMARY OF THE INVENTION

The present invention relates to the molecules disclosed in claims 1 to 4.

The present application discloses perfume raw materials, perfume delivery systems and consumer products comprising such perfume raw materials and/or such perfume delivery systems, as well as processes for making and using such perfume raw materials, perfume delivery systems and consumer products.

DETAILED DESCRIPTION OF THE INVENTION

Definitions

As used herein "consumer product" means baby care, beauty care, fabric & home care, family care, feminine care, health care, snack and/or beverage products or devices generally intended to be used or consumed in the form in which it is sold. Such products include but are not limited to diapers, bibs, wipes; products for and/or methods relating to treating hair (human, dog, and/or cat), including, bleaching, coloring, dyeing, conditioning, shampooing, styling; deodorants and antiperspirants; personal cleansing; cosmetics; skin care including application of creams, lotions, and other topically applied products for consumer use including fine fragrances; and shaving products, products for and/or methods relating to treating fabrics, hard surfaces and any other surfaces in the area of fabric and home care, including: air care including air fresheners and scent delivery systems, car care, dishwashing, fabric conditioning (including softening and/or freshening), laundry detergency, laundry and rinse additive and/or care, hard surface cleaning and/or treatment including floor and toilet bowl cleaners, and other cleaning for consumer or institutional use; products and/or methods relating to bath tissue, facial tissue, paper handkerchiefs, and/or paper towels; tampons, feminine napkins; products and/or methods relating to oral care including toothpastes, tooth gels, tooth rinses, denture adhesives, tooth whitening; over-the-counter health care including cough and cold remedies, pain relievers, RX pharmaceuticals, pet health and nutrition; processed food products intended primarily for consumption between customary meals or as a meal accompaniment (non-limiting examples include potato chips, tortilla chips, popcorn, pretzels, corn chips, cereal bars, vegetable chips or crisps, snack mixes, party mixes, multigrain chips, snack crackers, cheese snacks, pork rinds, corn snacks, pellet snacks, extruded snacks and bagel chips); and coffee.

As used herein, the term "cleaning and/or treatment composition" is a subset of consumer products that includes, unless otherwise indicated, beauty care, fabric & home care products. Such products include, but are not limited to, products for treating hair (human, dog, and/or cat), including, bleaching, coloring, dyeing, conditioning, shampooing, styling; deodorants and antiperspirants; personal cleansing; cosmetics; skin care including application of creams, lotions, and other topically applied products for consumer use including fine fragrances; and shaving products, products for

treating fabrics, hard surfaces and any other surfaces in the area of fabric and home care, including: air care including air fresheners and scent delivery systems, car care, dishwashing, fabric conditioning (including softening and/or freshening), laundry detergency, laundry and rinse additive and/or care, hard surface cleaning and/or treatment including floor and toilet bowl cleaners, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, including antibacterial hand-wash types, cleaning bars, mouthwashes, denture cleaners, dentifrice, car or carpet shampoos, bathroom cleaners including toilet bowl cleaners; hair shampoos and hair-rinses; shower gels, fine fragrances and foam baths and metal cleaners; as well as cleaning auxiliaries such as bleach additives and "stain-stick" or pre-treat types, substrate-laden products such as dryer added sheets, dry and wetted wipes and pads, nonwoven substrates, and sponges; as well as sprays and mists all for consumer or/and institutional use; and/or methods relating to oral care including toothpastes, tooth gels, tooth rinses, denture adhesives, tooth whitening.

As used herein, the term "fabric and/or hard surface cleaning and/or treatment composition" is a subset of cleaning and treatment compositions that includes, unless otherwise indicated, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, including antibacterial hand-wash types, cleaning bars, car or carpet shampoos, bathroom cleaners including toilet bowl cleaners; and metal cleaners, fabric conditioning products including softening and/or freshening that may be in liquid, solid and/or dryer sheet form; as well as cleaning auxiliaries such as bleach additives and "stain-stick" or pre-treat types, substrate-laden products such as dryer added sheets, dry and wetted wipes and pads, nonwoven substrates, and sponges; as well as sprays and mists. All of such products which were applicable may be in standard, concentrated or even highly concentrated form even to the extent that such products may in certain aspect be non-aqueous.

As used herein, the term "oral care composition" is a product, which in the ordinary course of usage, is not intentionally swallowed for purposes of systemic administration of particular therapeutic agents, but is rather retained in the oral cavity for a time sufficient to contact substantially all of the dental surfaces and/or oral tissues for purposes of oral activity. The oral care composition may be in various forms including toothpaste, dentifrice, tooth gel, subgingival gel, mouthrinse, mousse, foam, mouthspray, lozenge, chewable tablet, chewing gum or denture product. The oral care composition may also be incorporated onto strips or films for direct application or attachment to oral surfaces. The term "dentifrice", as used herein, includes paste, gel, or liquid formulations unless otherwise specified.
The dentifrice composition may be a single phase composition or may be a combination of two or more separate dentifrice compositions. The dentifrice composition may be in any desired form, such as deep striped, surface striped, multilayered, having a gel surrounding a paste, or any combination thereof. Each dentifrice composition in a dentifrice comprising two or more separate dentifrice compositions may be contained in a physically separated compartment of a dispenser and dispensed side-by-side.

As used herein, articles such as "a" and "an" when used in a claim, are understood to mean one or more of what is claimed or described.

As used herein, the terms "include", "includes" and "including" are meant to be non-limiting.

As used herein, the term "solid" includes granular, powder, bar and tablet product forms.

As used herein, the term "fluid" includes liquid, gel, paste and gas product forms.

As used herein, the term "situs" includes paper products, fabrics, garments, hard surfaces, hair and skin.

As used herein, "perfume raw materials" include molecules that can serve the purposes of providing odour and/or a sensation such as cooling.

Unless otherwise noted, all component or composition levels are in reference to the active portion of that component or composition, and are exclusive of impurities, for example, residual solvents or by-products, which may be present in commercially available sources of such components or compositions.

All percentages and ratios are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Table 1 Molecules (also known as "PRMs")

| Suitable molecules include the PRMs listed in Table 1 below and stereoisomers thereof. | | | |
|---|---|---|---|
| Number | Chemical Structure | IUPAC name | Character description |
| 1 | | 6,6,10-trimethylundec-9-en-5-one | green (geranium), vegetable, violet |
| 2 | | 2-methyl-5-(4-methylpent-3-en-1-yl)furan | herbal, anisic, floral |
| 3 | | 3-isopropyl-3,6-dimethylhept-5-en-2-one | herbal, aromatic |
| 4 | | (*E*,*Z*)-3,3,7-trimethyloct-6-en-2-one oxime | green, earthy |
| 5 | | 2-(((1*R*,5*S*)-6,6-dimethylbicyclo[ 3.1.1] hept-2-en-2-yl)methyl)-5-methylfuran | woody, terpenic |
| 6 | | 4-((1*R*,5*S*)-6,6-dimethylbicyclo[ 3.1.1]hept-2-en-2-yl)butan-1-ol | woody, earthy |
| 7 | | 2-(cyclohex-2-en-1-(*R*,*S*)-yl)-5-methylfuran | green (chervil), herbal |
| 8 | | methyl 8-methylnon-6-enoate | fruity |
| 9 | | 5-(but-2-enoyl)-4,4,6-trimethylcyclohe x-2-en-1-yl acetate | apple - rose ketone smell |
| 10 | | 1-(1-(4-methylpent-3-en-1-yl)cyclobutyl)pr opan-1-one | fruity (pineapple, currant - grenadine), minty |
| 11 | | 2-(*R*,*S*)-ethyl-2,6-dimethylhept-5-enenitrile | herbal (fennel), fruity |

(continued)

| Suitable molecules include the PRMs listed in Table 1 below and stereoisomers thereof. | | | |
|---|---|---|---|
| Number | Chemical Structure | IUPAC name | Character description |
| **12** | | 3-(*R*,*S*)-ethyl-3,7-dimethyloct-6-en-2-one | woody, herbal (lavender) |
| **13** | | 6-(*R*,*S*)-ethyl-6,10-dimethylundec-9-en-5-one | herbal, floral |
| **14** | | 3-(*R*,*S*)-ethyl-3,7-dimethyloct-6-en-2-(*R*, *S*)-ol | floral (lilac), clean |
| **15** | | 6-(*R*,*S*)-ethyl-6,10-dimethylundec-9-en-5-(*R*,*S*)-ol | floral, herbal, |
| **16** | | 2-(*R*,*S*)-ethyl-2,5-dimethylhex-4-enenitrile | herbal, minty (carway), spicy (celery) |
| **17** | | 3-(*R*,*S*)-ethyl-3,6-dimethylhept-5-en-2-one | citrus, fruity, fresh |
| **18** | | 3-(*R*,*S*)-ethyl-3,6-dimethylhept-5-en-2-ol | floral, herbal (lilac, pine), clean |
| **19** | | 6-(*R*,*S*)-ethyl-6,9-dimethyldec-8-en-5-one | woody, leathery |
| **20** | | 6-(*R*,*S*)-ethyl-6,9-dimethyldec-8-en-5-ol | floral (lily of the valley) |
| **21** | | (*R*,*S*)-3,4-dimethylcyclohe x-3-enecarbonitrile | almond |

(continued)

| Suitable molecules include the PRMs listed in Table 1 below and stereoisomers thereof. | | | |
|---|---|---|---|
| Number | Chemical Structure | IUPAC name | Character description |
| **22** | | 1-(*R*,*S*)-,3,4-trimethylcyclohe x-3-enecarbonitrile | herbal, spicy (nutty) |
| **23** | | 1-(1-(*R*,*S*),3,4-trimethylcyclohe x-3-en-1-yl) ethanone | fresh, citrus, herbal, woody |
| **24** | | 3,4-dimethyl-1-(*R*,*S*)-(3-methylbut-2-en-1-yl)cyclohex-3-enecarbonitrile | mussel, terpenic |
| **25** | | 1-(*R*,*S*)-(1-(*R*,*S*),3,4-trimethyl-cyclohex-3-en-1-yl)ethanol | clean, pine, floral (lily of the valley) |
| **26** | | 1-(3,4-dimethyl-1-(*R*,*S*)-(3-methylbut-2-en-1-yl)cyclohex-3-en-1-yl)ethanone | clean, floral, woody (pine), green |
| **27** | | 1-(1-(*R*,*S*),3,4-trimethylcyclohe x-3-en-1-yl) pentan-1-one | woody, spicy |
| **28** | | 3,4-dimethyl-1-(*R*,*S*)-(4-methylpent-3-en-1-yl)cyclohex-3-enecarbonitrile | green |
| **29** | | 4-methoxy-3-propoxybenzalde hyde | vanilla, caramel |
| **30** | | 3-isopropoxy-4-methoxybenzald ehyde | nutty, roasted |

(continued)

| Suitable molecules include the PRMs listed in Table 1 below and stereoisomers thereof. | | | |
|---|---|---|---|
| Number | Chemical Structure | IUPAC name | Character description |
| 31 | | 1-(3-ethoxy-4-hydroxyphenyl)p ropan-1-one | sweet vanilla, heliotropine |
| 32 | | 1-(3-ethoxy-4-hydroxyphenyl)p entan-1-one | sweet powder |
| 33 | | 4-hydroxy-3-propoxybenzalde hyde | sweet, medicinal, mint |
| 34 | | 1-(3-ethoxy-4-hydroxyphenyl)-3-methylbutan-1-one | sweet balsamic, buttery |
| 35 | | 1-(3-ethoxy-4-hydroxyphenyl)-2-methylbutan-1-one | sweet, almond, green |
| 36 | | 1-(3-ethoxy-4-hydroxyphenyl)-2,2-dimethylpropan-1-one | sweet, dusty |
| 37 | | 1-(3-ethoxy-4-hydroxyphenyl)-2-methylpropan-1-one | sweet, phenolic, woody |
| 38 | | 3-(cyclopropylmet hoxy)-4-hydroxybenzalde hyde | sweet, floral, anisic |
| 39 | | 3-isobutoxy-4-methoxybenzald ehyde | sweet |

(continued)

| Suitable molecules include the PRMs listed in Table 1 below and stereoisomers thereof. | | | |
|---|---|---|---|
| Numbe r | Chemical Structure | IUPAC name | Character description |
| **40** | | 1-(*R*,*S*)-ethyl-3,4-dimethyl-cyclohex-3-enecarbonitrile | spicy |
| **41** | | 1-(1-(*R*,*S*)-ethyl-3,4-dimethyl-cyclohex-3-en-1-yl)ethanone | floral, pine needles, fresh (lime) |
| **42** | | 1-(1-(*R*,*S*)-ethyl-3,4-dimethyl-cyclohex-3-en-1-yl)pentan-1-one | chewing gum (fruity) |
| **43** | | 1-(1-(*R*,*S*)-methyl-3,4-dimethyl-cyclohex-3-en-1-yl)pentan-1-ol | fruity, herbal, chamomile |
| **44** | | 1-(*R*,*S*)allyl-3,4-dimethylcyclohe enecarbonitrile | green, violet |
| **45** | | 3,4-dimethyl-1-(*R*,*S*)-propylcyclohex-3-enecarbonitrile | citrus, herbal |
| **46** | | 1-(1-(*R*,*S*)-allyl-3,4-dimethyl-cyclohex-3-en-1-yl)ethanone | herbal (lavender), citrus, sweet |
| **47** | | 1-(1-(*R*,*S*)-allyl-3,4-dimethyl-cyclohex-3-en-1-yl)ethanol | woody |

(continued)

| Suitable molecules include the PRMs listed in Table 1 below and stereoisomers thereof. | | | |
|---|---|---|---|
| Number | Chemical Structure | IUPAC name | Character description |
| **48** | | 1-(3,4-dimethyl-1-(*R*,*S*)-propyl-cyclohex-3-en-1-yl)ethanone | fresh, citrus, floral, bergamot |
| **49** | | 1-(3,4-dimethyl-1-(*R*,*S*)propyl-cyclohex-3-en-1-yl)ethanol | hay, carrotseed |
| **50** | | 3,4-dimethyl-1-(*R*,*S*)-propyl-cyclohex-3-enecarbaldehyde | citrus, aldehydic |
| **51** | | 1-(*R*,*S*)-allyl-3,4-dimethyl-cyclohex-3-enecarbaldehyde | mint, herbal, tea, violet |
| **52** | | 1-(*R*,*S*)-(3,4-dimethyl-1-(4-methylpent-3-enyl)cyclohex-3-enyl)ethanone | floral, green |
| **53** | | 1-(*R*,*S*)-isopropyl-3,4-dimethylcyclohe x-3-enecarbonitrile | spicy, clean linen |
| **54** | | 3-methyl-1-(1-(*R*,*S*),3,4-trimethylcyclohe x-3-enyl)butan-1-one | green, unripe fruit, malt (beer) |
| **55** | | 3,4-dimethyl-1-(*R*,*S*)-(3-methylbut-2-enyl) cyclohex-3-enecarbaldehyde | floral (geranium) |

(continued)

| Suitable molecules include the PRMs listed in Table 1 below and stereoisomers thereof. | | | |
|---|---|---|---|
| Number | Chemical Structure | IUPAC name | Character description |
| **56** | | 1-(*R*,*S*)-(3,4-dimethyl-1-(3-methylbut-2-enyl)cyclohex-3-enyl)ethanol | herbal, citrus |
| **57** | | 1-(1-(*R*,*S*),3,4-trimethylcyclohe x-3-enyl) propan-1-one | citrus, floral (fresia), herbal |
| **58** | | 1-1-(*R*,*S*)-ethyl-3,4-dimethyl-cyclohex-3-enyl)-3-methylbutan-1-one | fruity, floral, herbal |
| **59** | | 1-(1-(*R*,*S*)-ethyl-3,4-dimethyl-cyclohex-3-enyl)propan-1-one | herbal (coriander) |
| **60** | | 2-(*R*,*S*)-allyl-2,6-dimethylhept-5-enenitrile | celery, mussels, sea |
| **61** | | 2,2,5-trimethyl-5-(*R*,*S*)-(4-methylpent-3-en-1-yl)-1,3-dioxane | nutty, creamy |
| **62** | | *(R,S)*-isopropyl 3,4-dimethylcyclohe xanecarboxylate | fruity (banana) |
| **63** | | 3,3,6-trimethylheptan-2-*(R,S)*-ol | herbal (lilac), citrus |
| **64** | | *(R,S)*-propyl 3,4-dimethyl cyclohex-3-enecarboxylate | white floral |

(continued)

| Suitable molecules include the PRMs listed in Table 1 below and stereoisomers thereof. | | | |
|---|---|---|---|
| Number | Chemical Structure | IUPAC name | Character description |
| **65** | | *(R,S)*-propyl 3,4-dimethylcyclohe xanecarboxylate | fruity, floral |
| **66** | | *(R,S)*-butyl 3,4-dimethylcyclohe x-3-enecarboxylate | fruity, floral |
| **67** | | *(R,S)*-butyl 3,4-dimethylcyclohe xanecarboxylate | fruity, floral |
| **68** | HO | *(R,S)*-(1,3,4-trimethylcyclohe xyl)methanol | herbal, woody, clean |
| **69** | OH | *(R,S)*-1-(3,4-dimethyl-1-propylcyclohexy l) ethanol | terpenic, earthy |
| **70** | | *(R,S)*-methyl 3,4-dimethylcyclohe xanecarboxylate | fruity (pineapple) |
| **71** | | *(R,S)*-methyl 1,3,4-trimethylcyclohe xanecarboxylate | fruity (grapefruit) |
| **72** | | *(R,S)*-(3,4-dimethylcyclohe x-3-enyl)methyl acetate | fruity (pear, strawberry), chewing gum |

(continued)

| Suitable molecules include the PRMs listed in Table 1 below and stereoisomers thereof. | | | |
|---|---|---|---|
| Number | Chemical Structure | IUPAC name | Character description |
| **73** | | (1-*(R,S)*,3,4-trimethylcyclohe x-3-enyl) methyl acetate | spicy, acidic |
| **74** | | (1-*(R,S)*,3,4-trimethylcyclohe x-3-enyl) methyl formate | herbal |
| **75** | | *(R,S)-(*3,4-dimethylcyclohe x-3-enyl)methyl formate | aldehydic, fresh (mimosa) |
| **76** | HO | *(R,S)*-(3,4-dimethylcyclohe xyl)methanol | green (apple) |
| **77** | | *(R,S)*-(1,3,4-trimethyl cyclohexyl)meth yl formate | violet, floral |
| **78** | | *(R,S)*-(1,3,4-trimethyl cyclohexyl)meth yl acetate | fruity, herbal |
| **79** | | *(R,S)*-(3,4-dimethyl cyclohexyl)meth yl acetate | rose, fruity |
| **80** | | *(R,S)*-1-(1,3,4-trimethylcyclohe x-3-enyl) ethyl acetate | fruity, herbal, clean |

(continued)

| Suitable molecules include the PRMs listed in Table 1 below and stereoisomers thereof. | | | |
|---|---|---|---|
| Numbe r | Chemical Structure | IUPAC name | Character description |
| **81** | | *(R,S)*-1-(1,3,4-trimethylcyclohe xyl)ethyl acetate | balsamic, sweet |
| **82** | NC | 1-*(R,S)*-isopropylcyclohe x-3-enecarbonitrile | spicy |
| **83** | | methyl 2-(*R*,*S*)-methyl -5(propan-2-ylidene)-cyclopentanecar boxylate | fresh, peppermint |
| **84** | | 1-*(R,S)*-(1,3,4,6-*(R,S)*-tetra-methylcyclohex-3-enyl)ethanone | cedarwood |
| **85** | | 1-(1,6-(*R*,*S*)-dimethylcyclohe x-3-enyl) ethanone | woody, camphoraceo us |
| **86** | OH | (*R*,*S*)-1-(1,3,4,6-tetra-methylcyclohex-3-enyl)ethanol | lilac |
| **87** | | *(R,S)*-1-(1,2,4,5-tetramethylcyclo hexyl) ethanone | woody, fruity |
| **88** | OH | *(R,S)*-1-(1,2,4,5-tetramethylcyclo hexyl) ethanol | sweet, fruity |

(continued)

| Suitable molecules include the PRMs listed in Table 1 below and stereoisomers thereof. | | | |
|---|---|---|---|
| Numbe r | Chemical Structure | IUPAC name | Character description |
| **89** | | (2-(*R*,*S*)-methyl-5-(propan-2-ylidene) cyclopen tyl)methanol | quinoline |
| **90** | | (*R*,*S*)-(2-methyl-5-(propan-2-ylidene) cyclopen tyl)methyl formate | woody |
| **91** | | 1-(1,2-dimethyl cyclohexyl)ethan one | woody, earthy camphoraceo us |
| **92** | | (*R*,*S*)-(2-methyl-5-(propan-2-ylidene) cyclopen tyl)methyl acetate | woody, violet |
| **93** | | (*R*,*S*)-1-(1,6-dimethylcyclohe x-3-enyl) ethanol | earthy (moss) |
| **94** | | (*R,S*)-1-(1,3,4,6-tetramethyl cyclohex-3-enyl) ethyl formate | violet, woody |
| **95** | | (*R*,*S*)-1-(1,6-dimethylcyclohe x-3-enyl)ethyl formate | (soft) woody, spicy |
| **96** | | (*R*,*S*)-2-(2-methyl-5-(propan-2-ylidene) cyclopen tyl)propan-2-ol | woody, minty |
| **97** | | (*R*,*S*)-1-(1,2-dimethyl cyclohexyl)ethan ol | woody, earthy |

(continued)

| Suitable molecules include the PRMs listed in Table 1 below and stereoisomers thereof. | | | |
|---|---|---|---|
| Numbe r | Chemical Structure | IUPAC name | Character description |
| **98** | | 1,3,4,6,8-pentamethyl-2-oxa-bicyclo[2.2.2] oct ane | herbal, camphor |
| **99** | | (*R*,*S*)-1-(1,6-dimethylcyclohe x-3-enyl)ethyl acetate | violet, fruity |
| **100** | | methyl 2,2,5-trimethylhex-4-enoate | floral (chamomile), aromatic, citrus |
| **101** | | 2,2,5-trimethylhex-4-en-1-ol | lilac, clean |
| **102** | | methyl 2,2-dimethyl-3-m-tolylpropanoate | cyclamen, herbal |
| **103** | | 2,2,5-trimethylhex-4-enyl formate | citrus, gun powder |
| **104** | | 2,2-dimethyl-3-m-tolylpropyl formate | citrus, lemon |
| **105** | | *(R,S)*-ethyl 3,4-dimethylcyclohe x-3-enecarboxylate | fruity, herbal, spicy |
| **106** | | (R,S)-ethyl 3,4-dimethylcyclohe xanecarboxylate | fruity (chewing gum), pineapple - strawberry - red apple |
| **107** | | 2,2,5-trimethylhex-4-enyl acetate | citrus, bergamot |
| **108** | | methyl 2,2-dimethyl-3-p-tolylpropanoate | mushroom, chervil |

(continued)

| Suitable molecules include the PRMs listed in Table 1 below and stereoisomers thereof. | | | |
|---|---|---|---|
| Numbe r | Chemical Structure | IUPAC name | Character description |
| **109** | | methyl 2,2-dimethyl-3-o-tolylpropanoate | saffron, woody |
| **110** | | 2,3,3-trimethyl-4-m-tolylbutan-2-ol | dark chocolate |
| **111** | | 2,2-dimethyl-3-m-tolylpropyl acetate | citrus |
| **112** | | 2,2-dimethyl-3-o-tolylpropan-1-ol | disinfectant (toilet bowl cleaner) |
| **113** | | isobutyl 3,4-dimethylcyclohe x-3-enecarboxylate | green, garlic, fruity, citrus |
| **114** | | isobutyl 3,4-dimethylcyclohe xanecarboxylate | fruity, butyric |
| **115** | | 2,2-dimethyl-3-p-tolylpropan-1-ol | violet, minty |
| 116 | | tert-butyl 3,4-dimethylcyclohe x-3-enecarboxylate | herbal, aromatic |
| **117** | | *(R,S)*-tert-butyl 3,4-dimethylcyclohe xanecarboxylate | clean, ozonic |

(continued)

| Suitable molecules include the PRMs listed in Table 1 below and stereoisomers thereof. | | | |
|---|---|---|---|
| Number | Chemical Structure | IUPAC name | Character description |
| **118** | | 4,5-dimethyl-1,2,3,6-tetrahydro-[1,1'-biphenyl]-2-carbaldehyde | floral, fruity, cherry |
| **119** | | 3-(*R*,*S*)-allyl-3,7-dimethyloct-6-en-2-one | floral |
| **120** | | 3-(*R*,*S*)-allyl-3,7-dimethyloct-6-en-2-(*R*, *S*)-ol | floral |
| **121** | | 3,3,8,9-tetramethyl-2,4-dioxaspiro[5.5]undec-8-ene | green |
| **122** | | methyl 1-(*R*,*S*),3,4-trimethyl-cyclohex-3-enecarboxylate | fresh, citrus, herbal (bergamot) |
| **123** | | methyl 3,4-dimethylcyclohe x-3-ene-(*R*, *S*)-carboxylate | banana skin |
| **124** | | (3,4-dimethyl-(*R*,*S*)-cyclohex-3-enyl) methanol | floral (green lily) |
| **125** | | 2-(3,4-dimethylcyclohe x-3-enyl)-(*R*, *S*)-propan-2-ol | spicy (fenugreek) |

(continued)

| Suitable molecules include the PRMs listed in Table 1 below and stereoisomers thereof. | | | |
|---|---|---|---|
| Number | Chemical Structure | IUPAC name | Character description |
| **126** | | methyl 1-(*R*,*S*)-ethyl-3,4-dimethyl-cyclohex-3-enecarboxylate | fresh, fruity, floral |
| **127** | HO | (1-(*R*,*S*)-ethyl-3,4-dimethyl-cyclohex-3-enyl)methanol | floral, lilac |
| **128** | OH | 1-(1-(*R*,*S*)-ethyl-3,4-dimethylcyclohe x-3-enyl)ethanol | floral, lilac |
| **129** | HO | (1-(*R*,*S*)-isopropyl-3,4-dimethyl-cyclohex-3-enyl)methanol | floral, aromatic |
| **130** | | methyl 3,4-dimethyl-1-(*R*, *S*)-propylcyclohex-3-enecarboxylate | violet, fruity, herbal |
| **131** | HO | (1-(*R*,*S*)-,3,4-trimethylcyclohe x-3-enyl) methanol | woody |
| **132** | HO | (3,4-dimethyl-1-(*R*,*S*)-propylcyclohex-3-enyl)methanol | terpenic, pine, woody |
| **133** | | methyl 3,4-dimethyl-1-(*R*,*S*)-(3-methylbut-2-enyl)cyclohex-3-enecarboxylate | citronella, clean |

(continued)

| Suitable molecules include the PRMs listed in Table 1 below and stereoisomers thereof. | | | |
|---|---|---|---|
| Numbe r | Chemical Structure | IUPAC name | Character description |
| **134** | | (3,4-dimethyl-1-(*R*,*S*)-(3-methylbut-2-enyl) cyclohex-3-enyl)methanol | herbal, woody, smoky |
| **135** | | (1-(*R*,*S*)-allyl-3,4-dimethyl-cyclohex-3-enyl) methanol | pine, indolic |
| **136** | | methyl 1-(*R*,*S*)-isopropyl-3,4-dimethylcyclohe x-3-enecarboxylate | violet |
| **137** | | methyl 1-(*R*,*S*)-allyl-3,4-dimethyl-cyclohex-3-enecarboxylate | green cucumber |
| **138** | | 2-(1-(R,S)-,3,4-trimethylcyclohe x-3-enyl) propan-2-ol | floral |
| **139** | | N,1-(*R*,*S*),3,4-tetramethyl-cyclohex-3-enecarboxamide | solventy, fruity |
| **140** | | 3,3,6-trimethylhept-5-en-2-one | citrus, fresh, grapefruit |
| **141** | | 3,3,6-trimethylhept-5-en-2-(*R*,*S*)-ol | lime, lilac |
| **142** | | 2,2,5,5,6-(*R*,*S*)-pentamethyl-tetrahydro-2H-pyran | camphor, green |

(continued)

| Suitable molecules include the PRMs listed in Table 1 below and stereoisomers thereof. | | | |
|---|---|---|---|
| Number | Chemical Structure | IUPAC name | Character description |
| **143** | | 1-*(R,S)*-butyl-3,4-dimethyl-cyclohex-3-enecarbonitrile | floral (rose, violet), herbal |
| **144** | | 1-(1-*(R,S)*-butyl-3,4-dimethyl-cyclohex-3-enyl)ethanone | spicy |
| **145** | | 1-(1-*(R,S)*-butyl-3,4-dimethylcyclohe x-3-enyl)ethanol | violet, calamus |
| **146** | | *(R,S)*-isopropyl 3,4-dimethyl-cyclohex-3-enecarboxylate | white floral, fresh, aldehydic |

The PRMs disclosed in Table 1 above (*a.k.a.*, molecules - as referred to in the Examples section) may provide one or more of the following benefits at levels that Applicants believe are unexpected in view of PRMs in general: a cooling sensation, neat product odor; wet fabric odor when applied to a fabric; dry fabric odor when applied to a fabric; reduced leakage from an encapsulate, including an encapsulate such as a perfume microcapsule; increased head space versus neat oil in certain perfume delivery technologies; odor when used in a matrix perfume delivery that is applied to a package; neat product odor when applied to a cleaning and/or treatment composition; fine fragrance composition odor when used in a fine fragrance; dry hair odor when a composition comprising such a PRM is applied to hair; PRM bloom from a solution comprising such a PRM; and new PRM character when applied to a situs. Confirmation of such benefits can be obtained by applying standard test methodologies detailed herein. The PRMs and stereoisomers of such PRMs disclosed in Table 1 above can be made in accordance with the teachings detailed in the present specification.

PRMs disclosed herein may have the structure of Table 1 molecules 1, 2, 3, 4, 5, 10, 11, 12, 13, 14, 15, 16, 17, 18, 19, 20, 22, 24, 26, 27, 28, 34, 35, 36, 37, 40, 41, 42, 43, 44, 45, 46, 47, 48, 49, 50, 51, 52, 53, 54, 55, 56, 57, 58, 59, 60, 61, 62, 64, 65, 67, 69, 70, 71, 73, 74, 75, 77, 79, 80, 81, 82, 84, 86, 87, 88, 93, 94, 95, 96, 97, 98, 99, 102, 104, 105, 106, 110, 111, 113, 114, 116,117, 119, 120, 121, 126, 127, 128, 129, 130, 132, 133, 134, 135, 136, 137, 139, 142, 143, 144, 145 and 146 and stereoisomers thereof.

In one aspect, a perfume or sensate composition comprising, based on total perfume weight, from about 0.01% to about 50%, from about 0.1% to about 15%, from about 0.1% to about 10% or even from about 0.5% to about 10% of one or more molecules selected from the molecules disclosed in claims 2 and 4 and mixtures thereof; and an optional solvent is disclosed.

In another aspect, the PRMs disclosed in Table 1 selected from the molecules disclosed in claims 1 to 4 are suitable for use, as defined by the present specification, in consumer products at levels, based on total consumer product weight, of from about 0.0001% to about 25%, preferably from about 0.0005% to about 10%, more preferably from about 0.001% to about 5%, more preferably from about 0.005% to about 2.5%, or most preferably from 0.01% to about 1%. Such PRMs and stereoisomers thereof may be used in various combinations in the aforementioned consumer products. Preferably, a consumer product may comprise more PRMs selected from Table 1 molecules 1, 2, 3, 4, 5, 6, 7, 8, 10, 11, 12, 13, 14, 15, 16, 17, 18, 19, 20, 21, 22, 23, 24, 25, 26, 27, 28, 31, 32, 34, 35, 36, 37, 38, 40, 41, 42, 43, 44, 45,

46, 47, 48, 49, 50, 51, 52, 53, 54, 55, 56, 57, 58, 59, 60, 61, 62, 63, 64, 65, 66, 67, 69, 70, 71, 73, 74, 75, 76, 77, 79, 80, 81, 82, 84, 85, 86, 87, 88, 91, 92, 93, 94, 95, 96, 97, 98, 99, 100, 102, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145 and 146 and stereoisomers thereof.

In another aspect, the PRMs selected from the molecules disclosed in claims 1 to 4 are suitable for use, as defined by the present specification, in cleaning and/or treatment compositions at levels, based on total cleaning and treatment products weight of from about 0.0001% to about 25%, preferably from about 0.0005% to about 10%, more preferably from about 0.001% to about 5%, more preferably from about 0.005% to about 2.5%, or most preferably from 0.01% to about 1%. Such PRMs and stereoisomers thereof may be used in various combinations in the aforementioned cleaning and/ treatment compositions. Preferably, a cleaning and/or treatment composition may comprise more PRMs selected from Table 1 molecules 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 84, 85, 86, 87, 88, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145 and 146; and stereoisomers thereof.

In another aspect, the PRMs selected from the molecules disclosed in claims 1 to 4 are suitable for use, as defined by the present specification, in fabric and/or hard surface cleaning and/or treatment compositions at levels, based on total fabric and/or hard surface cleaning and/or treatment composition weight of from about 0.00001% to about 25%, preferably from 0.00005% to about 10%, more preferably from 0.0001% to about 5%, more preferably from 0.0005% to about 1.0%, or most preferably from 0.001% to about 0.5%. Such PRMs and stereoisomers thereof may be used in various combinations in the aforementioned fabric and/or hard surface cleaning and/or treatment compositions. Preferably, a fabric and/or hard surface cleaning and/or treatment composition may comprise more PRMs selected from Table 1 molecules 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 84, 85, 86, 87, 88, 91, 92, 93, 94, 95, 96, 97, 98, 99, 100, 101, 102, 103, 104, 105, 106, 107, 108, 109, 110, 111, 112, 113, 114, 115, 116, 117 , 118, 119, 120, 121, 122, 123, 124, 125, 126, 127, 128, 129, 130, 131, 132, 133, 134, 135, 136, 137, 138, 139, 140, 141, 142, 143, 144, 145 and 146; and stereoisomers thereof

In another aspect, a detergent that may comprise the same level of the PRMs as disclosed for the aforementioned fabric and hard surface cleaning and/or treatment compositions is disclosed. The detergent may comprises one or more PRMs selected from the molecules disclosed in claims 1 to 4 .

In another aspect, the PRMs selected from the molecules disclosed in claims 1 to 4 are suitable for use in highly compacted consumer products, including highly compacted fabric and hard surface cleaning and/or treatment compositions. The PRMs selected from the molecules disclosed in claims 1 to 4 may be employed in solid or fluid highly compacted detergents at levels of from about 0.00001% to about 25%, preferably from 0.00005% to about 10%, more preferably from 0.0001% to about 5%, more preferably from 0.0005% to about 1.0%, or most preferably from 0.001% to about 0.5%, based on total composition weight. Such PRMs and stereoisomers thereof may be used in various combinations in the aforementioned highly compacted detergent compositions. Such highly compact detergents typically comprise a higher than normal percentage of active ingredients. Preferably, a highly compacted detergent may comprise more PRMs selected from Table 1 Nos. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 84, 85, 86, 87, 88, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145 and 146; and stereoisomers thereof.

Perfume Delivery Systems

Certain perfume delivery systems, methods of making certain perfume delivery systems and the uses of such perfume delivery systems are disclosed in USPA 2007/0275866 A1. Such perfume delivery systems include:

I. Polymer Assisted Delivery (PAD): This perfume delivery technology uses polymeric materials to deliver perfume materials. Classical coacervation, water soluble or partly soluble to insoluble charged or neutral polymers, liquid crystals, hot melts, hydrogels, perfumed plastics, microcapsules, nano- and micro-latexes, polymeric film formers, and polymeric absorbents, polymeric adsorbents, etc. are some examples. PAD includes but is not limited to:

a.) Matrix Systems: The fragrance is dissolved or dispersed in a polymer matrix or particle. Perfumes, for

example, may be 1) dispersed into the polymer prior to formulating into the product or 2) added separately from the polymer during or after formulation of the product. Diffusion of perfume from the polymer is a common trigger that allows or increases the rate of perfume release from a polymeric matrix system that is deposited or applied to the desired surface (situs), although many other triggers are know that may control perfume release. Absorption and/or adsorption into or onto polymeric particles, films, solutions, and the like are aspects of this technology. Nano- or micro-particles composed of organic materials (e.g., latexes) are examples. Suitable particles include a wide range of materials including, but not limited to polyacetal, polyacrylate, polyacrylic, polyacrylonitrile, polyamide, polyaryletherketone, polybutadiene, polybutylene, polybutylene terephthalate, polychloroprene, poly ethylene, polyethylene terephthalate, polycyclohexylene dimethylene terephthalate, polycarbonate, polychloroprene, polyhydroxyalkanoate, polyketone, polyester, polyethylene, polyetherimide, polyethersulfone, polyethylenechlorinates, polyimide, polyisoprene, polylactic acid, polymethylpentene, polyphenylene oxide, polyphenylene sulfide, polyphthalamide, polypropylene, polystyrene, polysulfone, polyvinyl acetate, polyvinyl chloride, as well as polymers or copolymers based on acrylonitrile-butadiene, cellulose acetate, ethylene-vinyl acetate, ethylene vinyl alcohol, styrene-butadiene, vinyl acetate-ethylene, and mixtures thereof.

"Standard" systems refer to those that are "pre-loaded" with the intent of keeping the pre-loaded perfume associated with the polymer until the moment or moments of perfume release. Such polymers may also suppress the neat product odor and provide a bloom and/or longevity benefit depending on the rate of perfume release. One challenge with such systems is to achieve the ideal balance between 1) in-product stability (keeping perfume inside carrier until you need it) and 2) timely release (during use or from dry situs). Achieving such stability is particularly important during in-product storage and product aging. This challenge is particularly apparent for aqueous-based, surfactant-containing products, such as heavy duty liquid laundry detergents. Many "Standard" matrix systems available effectively become "Equilibrium" systems when formulated into aqueous-based products. One may select an "Equilibrium" system or a Reservoir system, which has acceptable in-product diffusion stability and available triggers for release (e.g., friction). "Equilibrium" systems are those in which the perfume and polymer may be added separately to the product, and the equilibrium interaction between perfume and polymer leads to a benefit at one or more consumer touch points (versus a free perfume control that has no polymer-assisted delivery technology). The polymer may also be pre-loaded with perfume; however, part or all of the perfume may diffuse during in-product storage reaching an equilibrium that includes having desired perfume raw materials (PRMs) associated with the polymer. The polymer then carries the perfume to the surface, and release is typically via perfume diffusion. The use of such equilibrium system polymers has the potential to decrease the neat product odor intensity of the neat product (usually more so in the case of pre-loaded standard system). Deposition of such polymers may serve to "flatten" the release profile and provide increased longevity. As indicated above, such longevity would be achieved by suppressing the initial intensity and may enable the formulator to use more high impact or low odor detection threshold (ODT) or low Kovats Index (KI) PRMs to achieve FMOT benefits without initial intensity that is too strong or distorted. It is important that perfume release occurs within the time frame of the application to impact the desired consumer touch point or touch points. Suitable micro-particles and micro-latexes as well as methods of making same may be found in USPA 2005/0003980 A1. Matrix systems also include hot melt adhesives and perfume plastics. In addition, hydrophobically modified polysaccharides may be formulated into the perfumed product to increase perfume deposition and/or modify perfume release. All such matrix systems, including for example polysaccharides and nanolatexes may be combined with other PDTs, including other PAD systems such as PAD reservoir systems in the form of a perfume microcapsule (PMC). Polymer Assisted Delivery (PAD) matrix systems may include those described in US Patent Applications 2004/0110648 A1.

Silicones are also examples of polymers that may be used as PDT, and can provide perfume benefits in a manner similar to the polymer-assisted delivery "matrix system". Such a PDT is referred to as silicone-assisted delivery (SAD). One may pre-load silicones with perfume, or use them as an equilibrium system as described for PAD. Functionalized silicones may also be used. Examples of silicones include polydimethylsiloxane and polyalkyldimethylsiloxanes. Other examples include those with amine functionality, which may be used to provide benefits associated with amine-assisted delivery (AAD) and/or polymer-assisted delivery (PAD) and/or amine-reaction products (ARP).

b.) Reservoir Systems: Reservoir systems are also known as a core-shell type technology, or one in which the fragrance is surrounded by a perfume release controlling membrane, which may serve as a protective shell. The material inside the microcapsule is referred to as the core, internal phase, or fill, whereas the wall is sometimes called a shell, coating, or membrane. Microparticles or pressure sensitive capsules or microcapsules are examples of this technology. Microcapsules of the current invention are formed by a variety of procedures that include, but are not limited to, coating, extrusion, spray-drying, interfacial, in-situ and matrix polymerization. The possible shell materials vary widely in their stability toward water. Among the most stable are polyoxymethyleneurea (PMU)-based materials, which may hold certain PRMs for even long periods of time in aqueous

solution (or product). Such systems include but are not limited to urea-formaldehyde and/or melamine-formaldehyde. Stable shell materials include polyacrylate-based materials obtained as reaction product of an oil soluble or dispersible amine with a multifunctional acrylate or methacrylate monomer or oligomer, an oil soluble acid and an initiator, in presence of an anionic emulsifier comprising a water soluble or water dispersible acrylic acid alkyl acid copolymer, an alkali or alkali salt. Gelatin-based microcapsules may be prepared so that they dissolve quickly or slowly in water, depending for example on the degree of cross-linking. Many other capsule wall materials are available and vary in the degree of perfume diffusion stability observed. Without wishing to be bound by theory, the rate of release of perfume from a capsule, for example, once deposited on a surface is typically in reverse order of in-product perfume diffusion stability. As such, urea-formaldehyde and melamine-formaldehyde microcapsules for example, typically require a release mechanism other than, or in addition to, diffusion for release, such as mechanical force (e.g., friction, pressure, shear stress) that serves to break the capsule and increase the rate of perfume (fragrance) release. Other triggers include melting, dissolution, hydrolysis or other chemical reaction, electromagnetic radiation, and the like. The use of pre-loaded microcapsules requires the proper ratio of in-product stability and in-use and/or on-surface (on-situs) release, as well as proper selection of PRMs. Microcapsules that are based on urea-formaldehyde and/or melamine-formaldehyde are relatively stable, especially in near neutral aqueous-based solutions. These materials may require a friction trigger which may not be applicable to all product applications. Other microcapsule materials (e.g., gelatin) may be unstable in aqueous-based products and may even provide reduced benefit (versus free perfume control) when in-product aged. Scratch and sniff technologies are yet another example of PAD.

II. Molecule-Assisted Delivery (MAD): Non-polymer materials or molecules may also serve to improve the delivery of perfume. Without wishing to be bound by theory, perfume may non-covalently interact with organic materials, resulting in altered deposition and/or release. Non-limiting examples of such organic materials include but are not limited to hydrophobic materials such as organic oils, waxes, mineral oils, petrolatum, fatty acids or esters, sugars, surfactants, liposomes and even other perfume raw material (perfume oils), as well as natural oils, including body and/or other soils. Perfume fixatives are yet another example. In one aspect, non-polymeric materials or molecules have a CLogP greater than about 2. Molecule-Assisted Delivery (MAD) may also include those described in USP 7,119,060.

III. Fiber-Assisted Delivery (FAD): The choice or use of a situs itself may serve to improve the delivery of perfume. In fact, the situs itself may be a perfume delivery technology. For example, different fabric types such as cotton or polyester will have different properties with respect to ability to attract and/or retain and/or release perfume. The amount of perfume deposited on or in fibers may be altered by the choice of fiber, and also by the history or treatment of the fiber, as well as by any fiber coatings or treatments. Fibers may be woven and non-woven as well as natural or synthetic. Natural fibers include those produced by plants, animals, and geological processes, and include but are not limited to cellulose materials such as cotton, linen, hemp jute, flax, ramie, and sisal, and fibers used to manufacture paper and cloth. Fiber-Assisted Delivery may consist of the use of wood fiber, such as thermomechanical pulp and bleached or unbleached kraft or sulfite pulps. Animal fibers consist largely of particular proteins, such as silk, sinew, catgut and hair (including wool). Polymer fibers based on synthetic chemicals include but are not limited to polyamide nylon, PET or PBT polyester, phenol-formaldehyde (PF), polyvinyl alcohol fiber (PVOH), polyvinyl chloride fiber (PVC), polyolefins (PP and PE), and acrylic polymers. All such fibers may be pre-loaded with a perfume, and then added to a product that may or may not contain free perfume and/or one or more perfume delivery technologies. In one aspect, the fibers may be added to a product prior to being loaded with a perfume, and then loaded with a perfume by adding a perfume that may diffuse into the fiber, to the product. Without wishing to be bound by theory, the perfume may absorb onto or be adsorbed into the fiber, for example, during product storage, and then be released at one or more moments of truth or consumer touch points.

IV. Amine Assisted Delivery (AAD): The amine-assisted delivery technology approach utilizes materials that contain an amine group to increase perfume deposition or modify perfume release during product use. There is no requirement in this approach to pre-complex or pre-react the perfume raw material(s) and amine prior to addition to the product. In one aspect, amine-containing AAD materials suitable for use herein may be non-aromatic; for example, polyalkylimine, such as polyethyleneimine (PEI), or polyvinylamine (PVAm), or aromatic, for example, anthranilates. Such materials may also be polymeric or non-polymeric. In one aspect, such materials contain at least one primary amine. This technology will allow increased longevity and controlled release also of low ODT perfume notes (e.g., aldehydes, ketones, enones) via amine functionality, and delivery of other PRMs, without being bound by theory, via polymer-assisted delivery for polymeric amines. Without technology, volatile top notes can be lost too quickly, leaving a higher ratio of middle and base notes to top notes. The use of a polymeric amine allows higher levels of top notes and other PRMS to be used to obtain freshness longevity without causing neat product odor to be more

intense than desired, or allows top notes and other PRMs to be used more efficiently. In one aspect, AAD systems are effective at delivering PRMs at pH greater than about neutral. Without wishing to be bound by theory, conditions in which more of the amines of the AAD system are deprotonated may result in an increased affinity of the deprotonated amines for PRMs such as aldehydes and ketones, including unsaturated ketones and enones such as damascone. In another aspect, polymeric amines are effective at delivering PRMs at pH less than about neutral. Without wishing to be bound by theory, conditions in which more of the amines of the AAD system are protonated may result in a decreased affinity of the protonated amines for PRMs such as aldehydes and ketones, and a strong affinity of the polymer framework for a broad range of PRMs. In such an aspect, polymer-assisted delivery may be delivering more of the perfume benefit; such systems are a subspecies of AAD and may be referred to as Amine-Polymer-Assisted Delivery or APAD. In some cases when the APAD is employed in a composition that has a pH of less than seven, such APAD systems may also be considered Polymer-Assisted Delivery (PAD). In yet another aspect, AAD and PAD systems may interact with other materials, such as anionic surfactants or polymers to form coacervate and/or coacervates-like systems. In another aspect, a material that contains a heteroatom other than nitrogen, for example sulfur, phosphorus or selenium, may be used as an alternative to amine compounds. In yet another aspect, the aforementioned alternative compounds can be used in combination with amine compounds. In yet another aspect, a single molecule may comprise an amine moiety and one or more of the alternative heteroatom moieties, for example, thiols, phosphines and selenols. Suitable AAD systems as well as methods of making same may be found in US Patent Applications 2005/0003980 A1.

V. Cyclodextrin Delivery System (CD): This technology approach uses a cyclic oligosaccharide or cyclodextrin to improve the delivery of perfume. Typically a perfume and cyclodextrin (CD) complex is formed. Such complexes may be preformed, formed in-situ, or formed on or in the situs. Without wishing to be bound by theory, loss of water may serve to shift the equilibrium toward the CD-Perfume complex, especially if other adjunct ingredients (e.g., surfactant) are not present at high concentration to compete with the perfume for the cyclodextrin cavity. A bloom benefit may be achieved if water exposure or an increase in moisture content occurs at a later time point. In addition, cyclodextrin allows the perfume formulator increased flexibility in selection of PRMs. Cyclodextrin may be pre-loaded with perfume or added separately from perfume to obtain the desired perfume stability, deposition or release benefit. Suitable CDs as well as methods of making same may be found in USPA 2005/0003980 A1.

VI. Starch Encapsulated Accord (SEA): The use of a starch encapsulated accord (SEA) technology allows one to modify the properties of the perfume, for example, by converting a liquid perfume into a solid by adding ingredients such as starch. The benefit includes increased perfume retention during product storage, especially under non-aqueous conditions. Upon exposure to moisture, a perfume bloom may be triggered. Benefits at other moments of truth may also be achieved because the starch allows the product formulator to select PRMs or PRM concentrations that normally cannot be used without the presence of SEA. Another technology example includes the use of other organic and inorganic materials, such as silica to convert perfume from liquid to solid. Suitable SEAs as well as methods of making same may be found in USP 6,458,754 B1.

VII. Inorganic Carrier Delivery System (ZIC): This technology relates to the use of porous zeolites or other inorganic materials to deliver perfumes. Perfume-loaded zeolite may be used with or without adjunct ingredients used for example to coat the perfume-loaded zeolite (PLZ) to change its perfume release properties during product storage or during use or from the dry situs. Suitable zeolite and inorganic carriers as well as methods of making same may be found in USPA 2005/0003980 A1. Silica is another form of ZIC. Another example of a suitable inorganic carrier includes inorganic tubules, where the perfume or other active material is contained within the lumen of the nano- or micro-tubules. In one aspect, the perfume-loaded inorganic tubule (or Perfume-Loaded Tubule or PLT) is a mineral nano- or micro-tubule, such as halloysite or mixtures of halloysite with other inorganic materials, including other clays. The PLT technology may also comprise additional ingredients on the inside and/or outside of the tubule for the purpose of improving in-product diffusion stability, deposition on the desired situs or for controlling the release rate of the loaded perfume. Monomeric and/or polymeric materials, including starch encapsulation, may be used to coat, plug, cap, or otherwise encapsulate the PLT. Suitable PLT systems as well as methods of making same may be found in USP 5,651,976.

VIII. Pro-Perfume (PP): This technology refers to perfume technologies that result from the reaction of perfume materials with other substrates or chemicals to form materials that have a covalent bond between one or more PRMs and one or more carriers. The PRM is converted into a new material called a pro-PRM (i.e., pro-perfume), which then may release the original PRM upon exposure to a trigger such as water or light. Pro-perfumes may provide enhanced perfume delivery properties such as increased perfume deposition, longevity, stability, retention, and the like. Pro-perfumes include those that are monomeric (non-polymeric) or polymeric, and may be pre-formed or may

be formed in-situ under equilibrium conditions, such as those that may be present during in-product storage or on the wet or dry situs. Nonlimiting examples of pro-perfumes include Michael adducts (e.g., beta-amino ketones), aromatic or non-aromatic imines (Schiff bases), oxazolidines, beta-keto esters, and orthoesters. Another aspect includes compounds comprising one or more beta-oxy or beta-thio carbonyl moieties capable of releasing a PRM, for example, an alpha, beta-unsaturated ketone, aldehyde or carboxylic ester. The typical trigger for perfume release is exposure to water; although other triggers may include enzymes, heat, light, pH change, autoxidation, a shift of equilibrium, change in concentration or ionic strength and others. For aqueous-based products, light-triggered pro-perfumes are particularly suited. Such photo-pro-perfumes (PPPs) include but are not limited to those that release coumarin derivatives and perfumes and/or pro-perfumes upon being triggered. The released pro-perfume may release one or more PRMs by means of any of the above mentioned triggers. In one aspect, the photo-pro-perfume releases a nitrogen-based pro-perfume when exposed to a light and/or moisture trigger. In another aspect, the nitrogen-based pro-perfume, released from the photo-pro-perfume, releases one or more PRMs selected, for example, from aldehydes, ketones (including enones) and alcohols. In still another aspect, the PPP releases a dihydroxy coumarin derivative. The light-triggered pro-perfume may also be an ester that releases a coumarin derivative and a perfume alcohol. In one aspect the pro-perfume is a dimethoxybenzoin derivative as described in USPA 2006/0020459 A1. In another aspect the pro-perfume is a 3', 5'-dimethoxybenzoin (DMB) derivative that releases an alcohol upon exposure to electromagnetic radiation. In yet another aspect, the pro-perfume releases one or more low ODT PRMs, including tertiary alcohols such as linalool, tetrahydrolinalool, or dihydromyrcenol. Suitable pro-perfumes and methods of making same can be found in US Patents 7,018,978 The PRMs disclosed in Table 1 and stereoisomers thereof are suitable for use in perfume delivery systems at levels, based on total perfume delivery system weight, of from 0.001% to about 50%, preferably from 0.005% to 30%, more preferably from 0.01% to about 10%, more preferably from 0.025% to about 5%, or most preferably from 0.025% to about 1%.

In another aspect, the perfume delivery systems disclosed herein are suitable for use in consumer products, cleaning and treatment compositions, fabric and hard surface cleaning and/or treatment compositions, detergents, and highly compacted consumer products, including highly compacted fabric and hard surface cleaning and/or treatment compositions (e.g., solid or fluid highly compacted detergents) at levels, based on total consumer product weight, from about 0.001% to about 20%, preferably from about 0.01% to about 10%, more preferably from about 0.05% to about 5%, most preferably from about 0.1% to about 0.5%.

In another aspect, the amount of PRMs selected from the molecules disclosed in claims 1 to 4 present in the perfume delivery systems, based on the total microcapsule and/or nanocapsule (Polymer Assisted Delivery (PAD) Reservoir System) weight, may be from about 0.1% to about 99%, preferably from 25% to about 95%, more preferably from 30 to about 90%, more preferably from 45% to about 90%, or most preferably from 65% to about 90%. Preferably, microcapsules and/or nanocapsules may comprise more PRMs selected from Table 1 molecules 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117 , 118, 119, 120, 121, 122, 123, 124, 125, 126, 127, 128, 129, 130, 131, 132, 133, 134, 135, 136, 137, 138, 139, 140, 141, 142, 143, 144, 145 and 146 ; stereoisomers of Table 1 molecules 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117 , 118, 119, 120, 121, 122, 123, 124, 125, 126, 127, 128, 129, 130, 131, 132, 133, 134, 135, 136, 137, 138, 139, 140, 141, 142, 143, 144, 145 and 146 and mixtures thereof.

In one aspect, the amount of total perfume based on total weight of starch encapsulates and starch agglomerates (Starch Encapsulated Accord (SEA)) ranges from 0.1% to about 99%, preferably from 25% to about 95%, more preferably from 30 to about 90%, more preferably from 45% to about 90%, or most preferably from 65% to about 90%. In one aspect, PRMs selected from the molecules disclosed in claims 1 to 4 are suitable for use in such starch encapsulates and starch agglomerates. Such PRMs may be used in combination in such starch encapsulates and starch agglomerates.

In another aspect, the amount of total perfume based on total weight of [cyclodextrin - perfume] complexes (Cyclodextrin (CD)) ranges from 0.1% to about 99%, preferably from 2.5% to about 75%, more preferably from 5% to about 60%, more preferably from 5% to about 50%, most preferably from 5% to about 25%. In one aspect, the PRMs selected from the molecules disclosed in claims 1 to 4 are suitable for use in such [cyclodextrin - perfume] complexes. Such PRMs may be used in combination in such [cyclodextrin - perfume] complexes.

In another aspect, the amount of total perfume based on total weight of Polymer Assisted Delivery (PAD) Matrix Systems (including Silicones) ranges from 0.1% to about 99%, preferably from 2.5% to about 75%, more preferably from 5% to about 60%, more preferably from 5% to about 50%, most preferably from 5% to about 25%. In one aspect,

the amount of total perfume based on total weight of a hot melt perfume delivery system/perfume loaded plastic Matrix System and ranges from 1% to about 99%, preferably from 2.5% to about 75%, more preferably from 5% to about 60%, more preferably from 5% to about 50%, most preferably from 10 % to about 50%. In one aspect, the PRMs selected from the molecules disclosed in claims 1 to 4 are suitable for use in such Polymer Assisted Delivery (PAD) Matrix Systems, including hot melt perfume delivery system/perfume loaded plastic Matrix Systems. Such PRMs may be used in various combinations in such Polymer Assisted Delivery (PAD) Matrix Systems (including hot melt perfume delivery system/perfume loaded plastic Matrix Systems).

In one aspect, the amount of total perfume based on total weight of Amine Assisted Delivery (AAD) (including Aminosilicones) ranges from 1% to about 99%, preferably from 2.5% to about 75%, more preferably from 5% to about 60%, more preferably from 5% to about 50%, most preferably from 5% to about 25%. In one aspect, the PRMs selected from the molecules disclosed in claims 1 to 4 are suitable for use in such Amine Assisted Delivery (AAD) systems. Such PRMs may be used in various combinations in such Amine Assisted Delivery (AAD) systems. Preferably, an Amine Assisted Delivery (AAD) system may comprise more PRMs selected from Table 1 molecules 1, 3, 10, 12, 13, 17, 19, 23, 26, 27, 29, 30, 31, 32, 33, 34, 35, 36, 37, 38, 39, 41, 42, 46, 48, 50, 51, 52, 54, 55, 57, 58, 59, 84, 85, 87, 91, 118, 119, 140 and 144; stereoisomers of Table 1 molecules 1, 3, 10, 12, 13, 17, 19, 23, 26, 27, 29, 30, 31, 32, 33, 34, 35, 36, 37, 38, 39, 41, 42, 46, 48, 50, 51, 52, 54, 55, 57, 58, 59, 84, 85, 87, 91, 118, 119, 140 and 144; and mixtures thereof.

PRM Nos. 1, 3, 10, 12, 13, 17, 19, 23, 26, 27, 31, 32, 34, 35, 36, 37, 41, 42, 46, 48, 52, 54, 57, 58, 59, 84, 85, 87, 91, 119, 140 and 144 are ketones. PRM Nos. 29, 30, 33, 38, 39, 50, 51, 55 and 118 are aldehydes.

A Pro-Perfume (PP) Amine Reaction Product (ARP) system may comprise one or more PRMs selected from Table 1 molecules 1, 3, 6, 10, 12, 13, 14, 15, 17, 18, 19, 20, 23, 25, 26, 27, 29, 30, 31, 32, 33, 34, 35, 36, 37, 38, 39, 41, 42, 43, 46, 47, 48, 49, 50, 51, 52, 54, 55, 56, 57, 58, 59, 63, 68, 69, 76, 84, 85, 86, 87, 88, 89, 91, 93, 96, 97, 101, 110, 112, 115, 118, 119, 120, 124, 125, 127, 128, 129, 131, 132, 134, 135, 138, 140, 141, 144 and 145; and mixtures thereof.

PRM Nos. 1, 3, 10, 12, 13, 17, 19, 23, 26, 27, 31, 32, 34, 35, 36, 37, 41, 42, 46, 48, 52, 54, 57, 58, 59, 84, 85, 87, 91, 119, 140 and 144 are ketones. PRM Nos. 29, 30, 33, 38, 39, 50, 51, 55 and 118 are aldehydes. PRMs Nos. 6, 14, 15, 18, 20, 25, 43, 47, 49, 56, 63, 68, 69, 76, 86, 88, 89, 93, 96, 97, 101, 110, 112, 115, 120,124,125,127,128,129,131,132,134,135,138,141 and 145 are alcohols.

In one aspect, the amount of total perfume based on total weight of Pro-Perfume (PP) Amine Reaction Product (ARP) system ranges from 0.1% to about 99%, preferably from about 1% to about 99%, more preferably from 5% to about 90%, more preferably from 10% to about 75%, more preferably from 20% to about 75%, most preferably from 25% to about 60%.

The perfume delivery technologies (a.k.a., perfume delivery systems) that are disclosed in the present specification may be used in any combination in any type of consumer product, cleaning and/or treatment composition, fabric and hard surface cleaning and/or treatment composition, detergent, and/or highly compact detergent.

## Perfumes

The PRMs disclosed in Table 1 may be used to formulate perfumes. Such perfumes are combinations of PRMs that may comprise a combination of Table 1 PRMs, or one or more Table 1 PRMs and one or more additional PRMs. When used in a perfume, the Table 1 PRMs may be employed, based on total perfume weight, at levels of from about 0.01% to about 50%, preferably from about 0.1% to about 15%, more preferably from about 0.1% to about 10% or most preferably from about 0.5% to about 10%. Such perfumes may be utilized in various applications, including being applied neat to a situs or used in a consumer product, cleaning and/or treatment composition, fabric and hard surface cleaning and/or treatment composition, detergent, and/or a highly compact detergent.

## Method of Use

In one aspect, a method of counteracting a malodor comprising contacting a situs with one or more molecules selected from the group consisting of molecules selected from claims 2 or 4 and mixtures thereof is disclosed. Preferably, said situs comprises said malodor. Preferably, said malodor comprises a thiol moiety.

## Adjunct Materials

For the purposes of the present invention, the non-limiting list of adjuncts illustrated hereinafter are suitable for use in the compositions detailed herein (e.g., consumer products, cleaning and/or treatment compositions, fabric and hard surface cleaning and/or treatment compositions, detergents, and/or a highly compact detergents). Such adjunct materials may be desirably incorporated in certain embodiments of the compositions, for example to assist or enhance performance of the composition, for treatment of the substrate to be cleaned, or to modify the aesthetics of the composition

as is the case with perfumes, colorants, dyes or the like. It is understood that such adjuncts are in addition to the components that are supplied via Applicants' perfumes and/or perfume systems detailed herein. The precise nature of these additional components, and levels of incorporation thereof, will depend on the physical form of the composition and the nature of the operation for which it is to be used.

Suitable adjunct materials include, but are not limited to, surfactants, builders, chelating agents, dye transfer inhibiting agents, dispersants, enzymes, and enzyme stabilizers, catalytic materials, bleach activators, polymeric dispersing agents, clay soil removal/anti-redeposition agents, brighteners, suds suppressors, dyes, additional perfume and perfume delivery systems, structure elasticizing agents, fabric softeners, carriers, hydrotropes, processing aids and/or pigments, metal salts, structurants or binders, anti-tartar agents, anti-caries agents, abrasives, fillers, humectants, breath agents, flavors, antibacterial agents. In addition to the disclosure below, suitable examples of such other adjuncts and levels of use are found in U.S. Patent No. 6,326,348 B1.

Each adjunct ingredient is not essential to Applicants' compositions. Thus, certain embodiments of Applicants' compositions may not contain one or more of the following adjuncts materials: bleach activators, surfactants, builders, chelating agents, dye transfer inhibiting agents, dispersants, enzymes, and enzyme stabilizers, catalytic metal complexes, polymeric dispersing agents, clay and soil removal/anti-redeposition agents, brighteners, suds suppressors, dyes, additional perfumes and perfume delivery systems, structure elasticizing agents, fabric softeners, carriers, hydrotropes, processing aids and/or pigments, metal salts, structurants or binders, anti-tartar agents, anti-caries agents, abrasives, fillers, humectants, breath agents, flavors, antibacterial agents. However, when one or more adjuncts are present, such adjuncts may be present as detailed below:

Surfactants - The compositions according to the present invention can comprise a surfactant or surfactant system wherein the surfactant can be selected from nonionic and/or anionic and/or cationic surfactants and/or ampholytic and/or zwitterionic and/or semi-polar nonionic surfactants. The surfactant is typically present at a level of from about 0.1%, from about 1%, or even from about 5% by weight of the cleaning compositions to about 99.9%, to about 80%, to about 35%, or even to about 30% by weight of the cleaning compositions.

Builders - The compositions of the present invention can comprise one or more detergent builders or builder systems. When present, the compositions will typically comprise at least about 1% builder, or from about 5% or 10% to about 80%, 50%, or even 30% by weight, of said builder. Builders include, but are not limited to, the alkali metal, ammonium and alkanolammonium salts of polyphosphates, alkali metal silicates, alkaline earth and alkali metal carbonates, aluminosilicate builders polycarboxylate compounds. ether hydroxypolycarboxylates, copolymers of maleic anhydride with ethylene or vinyl methyl ether, 1,3,5-trihydroxybenzene-2,4,6-trisulphonic acid, and carboxymethyl-oxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, oxydisuccinic acid, polymaleic acid, benzene 1,3,5-tricarboxylic acid, carboxymethyloxysuccinic acid, and soluble salts thereof.

Chelating Agents - The compositions herein may also optionally contain one or more copper, iron and/or manganese chelating agents. If utilized, chelating agents will generally comprise from about 0.1% by weight of the compositions herein to about 15%, or even from about 3.0% to about 15% by weight of the compositions herein.

Dye Transfer Inhibiting Agents - The compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. When present in the compositions herein, the dye transfer inhibiting agents are present at levels from about 0.0001%, from about 0.01%, from about 0.05% by weight of the cleaning compositions to about 10%, about 2%, or even about 1% by weight of the cleaning compositions.

Dispersants - The compositions of the present invention can also contain dispersants. Suitable water-soluble organic materials are the homo- or co-polymeric acids or their salts, in which the polycarboxylic acid may comprise at least two carboxyl radicals separated from each other by not more than two carbon atoms.

Enzymes - The compositions can comprise one or more detergent enzymes which provide cleaning performance and/or fabric care benefits. Examples of suitable enzymes include, but are not limited to, hemicellulases, peroxidases, proteases, cellulases, xylanases, lipases, phospholipases, esterases, cutinases, pectinases, keratanases, reductases, oxidases, phenoloxidases, lipoxygenases, ligninases, pullulanases, tannases, pentosanases, malanases, β-glucanases, arabinosidases, hyaluronidase, chondroitinase, laccase, and amylases, or mixtures thereof. A typical combination is a cocktail of conventional applicable enzymes like protease, lipase, cutinase and/or cellulase in

conjunction with amylase.

Enzyme Stabilizers - Enzymes for use in compositions, for example, detergents can be stabilized by various techniques. The enzymes employed herein can be stabilized by the presence of water-soluble sources of calcium and/or magnesium ions in the finished compositions that provide such ions to the enzymes.

Catalytic Metal Complexes - Applicants' compositions may include catalytic metal complexes. One type of metal-containing bleach catalyst is a catalyst system comprising a transition metal cation of defined bleach catalytic activity, such as copper, iron, titanium, ruthenium, tungsten, molybdenum, or manganese cations, an auxiliary metal cation having little or no bleach catalytic activity, such as zinc or aluminum cations, and a sequestrate having defined stability constants for the catalytic and auxiliary metal cations, particularly ethylenediaminetetraacetic acid, ethylenediaminetetra (methyl-enephosphonic acid) and water-soluble salts thereof. Such catalysts are disclosed in U.S. patent 4,430,243.

If desired, the compositions herein can be catalyzed by means of a manganese compound. Such compounds and levels of use are well known in the art and include, for example, the manganese-based catalysts disclosed in U.S. patent 5,576,282. Cobalt bleach catalysts useful herein are known, and are described, for example, in U.S. patents 5,597,936.

Compositions herein may also suitably include a transition metal complex of a macropolycyclic rigid ligand - abbreviated as "MRL". As a practical matter, and not by way of limitation, the compositions and cleaning processes herein can be adjusted to provide on the order of at least one part per hundred million of the benefit agent MRL species in the aqueous washing medium, and may provide from about 0.005 ppm to about 25 ppm, from about 0.05 ppm to about 10 ppm, or even from about 0.1 ppm to about 5 ppm, of the MRL in the wash liquor. Suitable transition-metals in the instant transition-metal bleach catalyst include manganese, iron and chromium. Suitable MRL's herein are a special type of ultra-rigid ligand that is cross-bridged such as 5,12-diethyl-1,5,8,12-tetraazabicyclo[6.6.2]hexa-decane. Suitable transition metal MRLs are readily prepared by known procedures, such as taught for example in and U.S. patent 6,225,464.

Methods of Use

Some of the consumer products disclosed herein can be used to clean or treat a situs *inter alia* a surface or fabric. Typically at least a portion of the situs is contacted with an embodiment of Applicants' composition, in neat form or diluted in a liquor, for example, a wash liquor and then the situs may be optionally washed and/or rinsed. In one aspect, a situs is optionally washed and/or rinsed, contacted with a composition according to the present invention and then optionally washed and/or rinsed. For purposes of the present invention, washing includes but is not limited to, scrubbing, and mechanical agitation. The fabric may comprise most any fabric capable of being laundered or treated in normal consumer use conditions. Liquors that may comprise the disclosed compositions may have a pH of from about 3 to about 11.5. Such compositions are typically employed at concentrations of from about 500 ppm to about 15,000 ppm in solution. When the wash solvent is water, the water temperature typically ranges from about 5 °C to about 90 °C and, when the situs comprises a fabric, the water to fabric ratio is typically from about 1:1 to about 30:1.

TEST METHODS

It is understood that the test methods that are disclosed in the Test Methods Section of the present application should be used to determine the respective values of the parameters of Applicants' invention as such invention is described and claimed herein.

(1) ClogP

The logP values of many perfume ingredients have been reported; for example, the Pomona92 database, available from Daylight Chemical Information Systems, Inc. (Daylight CIS), Irvine, California, contains many, along with citations to the original literature. However, the logP values are most conveniently calculated by the "CLOGP" program, also available from Daylight CIS. This program also lists experimental logP values when they are available in the Pomona92 database. The "calculated logP" (ClogP) is determined by the fragment approach of Hansch and Leo (cf., A. Leo, in Comprehensive Medicinal Chemistry, Vol. 4, C. Hansch, P. G. Sammens, J. B. Taylor and C. A. Ramsden, Eds., p. 295, Pergamon Press, 1990, incorporated herein by reference). The fragment approach is based on the chemical structure of each perfume ingredient, and takes into account the numbers and types of atoms, the atom connectivity, and chemical bonding. The ClogP values, which are the most reliable and widely used estimates for this physicochemical property, are preferably used instead of the experimental logP values in the selection of perfume ingredients which are

useful in the present invention.

(2) Boiling Point

The boiling point of perfume ingredients is measured according to standard test method ASTM D2887-04a, "Standard Test Method for Boiling Range Distribution of Petroleum Fractions by Gas Chromatography," (ASTM International, West Conshohocken, Pennsylvania, USA. Section 5.2 of that method notes: "Boiling range distributions obtained by this test method are essentially equivalent to those obtained by true boiling point (TBP) distillation (see Test Method D 2892). They are not equivalent to results from low efficiency distillations such as those obtained with Test Method D 86 or D 1160."

(3) Headspace Ratio

(a) Obtain a fragrance free consumer product formulation.
(b) Obtain fragrance microcapsules whose water content has been adjusted to achieve a perfume content of 25wt% in the aqueous slurry.
(c) Prepare Sample A by adding 2.0 grams of the fragrance microcapsule aqueous slurry to 95 grams of the fragrance free consumer product formulation. Then add 3.0 grams of deionized water to balance the formulation to 100 grams. Age this formulation for 1 week at 40 degrees Centigrade.
(d) Prepare Sample B by adding 0.50 grams of the neat fragrance to 95 grams of fragrance free consumer product formulation. Then add 4.5 grams of deionized water to balance the formulation to 100 grams. Age this formulation for 1 week at 40 degrees Centigrade.

The Headspace Ratio for determining perfume leakage from a perfume delivery system is defined as the headspace concentration of Sample A divided by the headspace concentration of Sample B, $\frac{H_{Sample_A}}{H_{Sample_B}}$, where $H_{Sample_A}$ is the headspace concentration of a consumer product formulation Sample A, and $H_{Sample_B}$ is the headspace concentration of a consumer product formulation Sample B.

The Headspace Ratio for determining perfume delivery efficiency from a perfume delivery system is defined as the headspace concentration of Sample B divided by the headspace concentration of Sample A, $\frac{H_{Sample_B}}{H_{Sample_A}}$, where $H_{Sample_A}$ is the headspace concentration of a consumer product formulation Sample A, and $H_{Sample_B}$ is the headspace concentration of a consumer product formulation Sample B.

Solid-Phase Micro-Extraction (SPME)-Gas Chromatography/Mass Spectrometry is used to measure the level of perfume raw materials in the headspace of products. 1.0 grams of the 1 week at 40 degrees Centigrade aged sample are placed into a clean 20 ml headspace vial and allowed to equilibrate for at least 2 hours at room temperature.

The samples are then analyzed using the MPS2-SMPE-GC-MS analysis system (GC-02001-0153, MSD-02001-0154, MPS2-02001-0155).

Apparatus:

1. 20 ml headspace vial
2. Timer.
3. Gas Chromatograph (GC): Agilent model 6890 with a CIS-4 injector (Gerstel, Mulheim, Germany) and MPS-2 Autosampler and TDU. For SPME analysis, we used the split/splitless injector (not the CIS-4 injector).
4. GC column: J&W DB-5 MS, 30 M x 0.25 mm ID, 1.0 □m film thickness obtained from J&W Scientific of Folsom,

California, USA.
5. Carrier gas, helium, 1.5 ml/min. flow rate.
6. The injector liner is a special SPME liner (0.75 mm ID) from Supelco.
7. The Detector is a model 5973 Mass Selective Detector obtained from Agilent Technologies, Inc., Wilmington, DE, USA having a source temperature of about 230 °C, and a MS Quad temperature of about 150 °C.

Analysis procedure:

1. Transfer sample to proper sample tray and proceed with SPME-GC-MS analysis.
2. Start sequence of sample loading and analysis. In this step, the sample is allowed to equilibrate for at least two hours on the auto sampler tray, then sampled directly from the tray. The SPME fiber assembly is DVB/CAR/PDMS (50/30 um, 24 ga, 1 cm length). Sampling time is 5 minutes.
3. Injector temperature is at 260C.
4. Then GC-MS analysis run is started. Desorption time is 5 minutes.
5. The following temperature program is used:

   i) an initial temperature of about 50 °C which is held for 3 minutes,
   ii) increase the initial temperature at a rate of about 6 °C/min until a temperature of about 250 °C is reached, then 25 °C/min to 275 °C, hold at about 275 °C for 4.67 minute.

6. Perfume compounds are identified using the MS spectral libraries of John Wiley & Sons and the National Institute of Standards and Technology (NIST), purchased and licensed through Hewlett Packard.
7. Chromatographic peaks for specific ions are integrated using the Chemstation software obtained from Agilent Technologies, Inc., Wilmington, DE, USA..
8. The ratio for each PRM is calculated by dividing the peak area for the perfume raw material in Sample A by the peak area in Sample B.
9. Each ratio is then weighted by that perfume raw material's weight composition in the perfume.
10. The Headspace Ratio is calculated as the sum of the individual perfume raw material ratios obtained in step 9.

(4) Perfume leakage can also be evaluated via% liquid-liquid extraction and gas chromatographic-mass spectrometric analysis

When determining the % perfume leakage from Perfume Microcapsules in liquid detergent, a fresh sample of liquid detergent with equal level of free perfume (without Perfume Microcapsules) must also be analyzed in parallel for reference.

1. Preparation of an internal standard solution

- Stock solution of tonalid : Weigh 70 mg tonalid and add 20 ml hexane p.a.
- Internal Standard Solution solution: Dilute 200 μl of stock solution in 20 ml hexane p.a.
- Mix to homogenize

2. Perfume extraction from liquid detergent without perfume microcapsules (reference)

- Weigh 2 g of liquid detergent product into an extraction vessel
- Add 2 ml of Internal Standard Solution and close vessel
- Extract perfume by gently turning the extraction vessel upside-down for 20 times (manually)
- Add spoon tip of Sodium Sulphate
- After separation of layers, immediately transfer hexane-layer into Gas Chromatograph auto sampler-vial and cap vial
- Inject splitless (1.5μl) into Gas Chromatograph injection-port
- Run Gas Chromatographic-Mass Spectrometric analysis

3. Perfume extraction from liquid detergent with perfume microcapsules

- Weigh 2 g of liquid detergent product into an extraction vessel
- Add 2 ml of Internal Standard Solution and close vessel
- Extract perfume by gently turning the extraction vessel upside-down for 20 times (manually)
- Add spoon tip of Sodium Sulphate
- After separation of layers, immediately transfer hexane-layer into Gas Chromatograph auto sampler-vial and cap vial
- Inject splitless (1.5μl) into Gas Chromatograph injection-port
- Run Gas Chromatographic-Mass Spectrometric analysis

4. Calculation

- The perfume leakage from capsules per individual Perfume Raw Material:

% perfume leakage = ((Area Perfume Raw Material caps x Area Internal Standard Solution ref x Weight ref) / (Area Internal Standard Solution caps x Area Perfume Raw Material ref x Weight caps)) x 100

(5) Odor Detection Threshold (OPT)

Determined using a gas chromatograph. The gas chromatograph is calibrated to determine the exact volume of material injected by the syringe, the precise split ratio, and the hydrocarbon response using a hydrocarbon standard of known concentration and chain length distribution. The air flow rate is accurately measured and, assuming the duration of human inhalation to last 12 seconds, the sampled volume is calculated. Since the precise concentration at the detector at any point in time is known, the mass per volume inhaled is known, and hence the concentration of material.
For example, to determine whether a material has a threshold below 50 parts per billion, solutions are delivered to the sniff port at the calculated concentration. A panelist sniffs the GC effluent and identifies the retention time when odor is noticed. The average among 6 panelists determines the threshold of notice ability. The necessary amount of analyte is injected into the column to achieve a 50 parts per billion concentration at the detector. Typical gas chromatograph parameters for determining odor detection thresholds are listed below:

GC: 5890 Series II with FID detector, 7673 Autosampler
Column: J&W Scientific DB-1
Length: 30 meters, 0.25millmeter inside diameter, 1 micrometer film thickness
Method:

- split injection: 17/1 split ratio
- Autosampler: 1.13 microliters per injection
- Column flow: 1.10 milliLiters per minute
- Air Flow : 345 milliLiters per minute
- Inlet Temperature : 245 degrees Centigrade
- Detector Temperature: 285 degrees Centigrade
- Initial Temperature = 50 degrees Centigrade, 5 degrees Centigrade per minute ramp rate, final temperature = 280 degrees Centigrade, Final time = 6 minutes
- Leading assumptions: 12 seconds per sniff, GC air adds to sample dilution

(6) Coolant screening on the TRPM8 receptor

HEK-23 (human embryonic kidney) cells stably transfected with human TRPM8 were grown in 15ml growth medium [high glucose DMEM (Dulbecco's Modification of Eagle's Medium) supplemented with 10% FBS (fetal bovine serum), 100 ug/ml Penicillin/streptomycin, 5μg/ml blasticindin, and 100 μg/ml zeocin) in a 75 CM 2 flask for 3 days at 37° C in a mammalian cell culture incubator set at 5% CO2. Cells were detached with addition of 2 ml of trypsin-EDTA

buffer (GIBCO® 25200, Invitrogen) for about 2-3 min. Trypsin was inactivated by addition of 8 ml growth medium. Cells were transferred to a 50 ml tube and centrifuged at 850 rpm for 3 minutes to remove medium. After centrifugation, a pellet of cells was formed in the bottom of the tube separating them from the supernatant solution. The supernatant was discarded and the cell pellet was suspended in 1 ml of fresh growth medium to which 5 ul (12.5 ug) of Fluo-4 AM (Molecular Probes, Inc.) calcium indicator was added and incubated for 30 min with gentle shaking. (Fluo-4is a fluorescent dye used for quantifying cellular Ca t concentrations in the 100 nM to 1 microM range.) At the end of 30 minutes, 45 ml of assay buffer [1 xHBSS (Hank's Balanced Salt Solution), 20 mM HEPES (4-(2-Hydroxyethyl)-1-piperazineethanesulfonic acid)] was added to wash cells and the resulting mixture was then centrifuged at 850 rpm for 3 minutes to remove excess buffer and Fluo-4 AM calcium indicator. The pellet cells were re-suspended in 10 ml assay buffer and 90 ul aliquots (-50,000 cells) per well delivered to a 96-well assay plate containing 10 ul of test compounds (1 mM in assay buffer, final concentration 100 uM) or buffer control and incubated at room temperature for 30 minutes. After 30 minutes, plate is placed into a fluorometric imaging plate reader (FLIPR 384 from Molecular Devices) and basal fluorescence recorded (excitation wave length 488 nm and emission wave length 510 nm). The FLIPR assay is an accepted method for detecting changes in intracellular calcium concentration. Then 20 ul of 37.5 uM of the compounds of the invention were tested as TRPM8 agonist in the assay buffer (final concentration 6.25 uM) was added and fluorescence recorded. For determining the direct effect of test compounds on TRPM8, fluorescence was measured immediately after addition of each compound.

Test Method - Malodor reduction Value

Selection and Training of Assessors

A. The assessors must be able to differentiate the sweat odour from an odourless solvent (dipropylene glycol=DPG). To do this, several cardboard smelling strips are immersed in a highly dilute solution (0.1% in DPG) of the 3-mercapto-3-methyl-hexan-1-ol. In addition, several smelling strips are immersed in DPG. Only those assessors who can perfectly differentiate the smelling strips in a test with 3-mercapto-3-methyl-hexan-1-ol -- 3-mercapto-3-methyl-hexan-1-ol -- DPG and DPG -- DPG -- 3-mercapto-3-methyl-hexan-1-ol take part in further tests.

B. Several concentrations of 3-mercapto-3-methyl-hexan-1-ol are then placed in a container with a volume of 7 1 filled with air. The samples are sorted by the assessors according to intensity, i.e. odour strength. The series of concentrations must be correctly recognised and evaluated by the assessors. Assessors who have passed both tests can take part in the raw material test described in the following.

Material Test Against Sweat Odour

The test materials examined are either individual perfume raw materials (PRMs) or perfume oils. The test materials are evaluated by the selected assessors in a predefined gaseous sample with regard to intensity and residual odour strength of the 3-mercapto-3-methyl-hexan-1-ol, a target malodor compound.

1 mu/l of the test material and 5 mu/l of the dilute 3-mercapto-3-methyl-hexan-1-ol (0.1% in DPG) respectively are placed in a container with a volume of 7 1 filled with air. The samples are kept at room temperature (20 deg. C.) for 15 h before evaluation. The individual samples are each evaluated by at least 8 assessors by smelling in comparison with a sample just with 3-mercapto-3-methyl-hexan-1-ol-solution. The mean value is then formed from the at least 8 values obtained for the respective raw material. The intensity of a test material describes the intensity of the test material perceived by odour by trained assessors, irrespective of the quality of the odour as a bad odour or pleasant odour. The stronger a test material smells the higher is the level of the intensity. The intensity is evaluated on a scale of 1 to 9. Level 1 means odourless, 9 means very strong odour detected. The term malodour reduction value describes in the present case the difference in intensity, i.e. the difference between the bad odour (malodour) of the malodour standard mixture and the test mixture.

The malodour standard mixture without test material, i.e., 3-mercapto-3-methyl-hexan-1-ol, receives intensity 6. The assessors are selected on the basis of their ability to reproducibly evaluate the strengths of odours. The assessors are trained before the series of tests to recognise the odour of 3-mercapto-3-methyl-hexan-1-ol.

EXAMPLES

While particular embodiments of the present invention have been illustrated and described, it would be obvious to those skilled in the art that various other changes and modifications can be made without departing from the scope of the invention. It is therefore intended to cover in the appended claims all such changes and modifications that are

within the scope of this invention.

Synthesis of Table 1 Molecule Number 11, 16, 22, 24, 28, 40, 44, 45, 53, 60, 82, 100, 102, 108 , 109, 126, 130, 133, 136, 137 and 143 or intermediates used for synthesis of Table 1 Molecule Number 1, 3, 4, 6, 10, 60, 63, 71, 73, 74, 77, 78 and 140:

1 eq. LDA, THF (0.5M)

1 eq. LDA, THF (0.5M)

1 eq. LDA, THF (0.5M)

| Compound Number | Nitrile | $R_1$ | $R_2$ | $R_3$ | $R_4$ | $R_5$ | X |
|---|---|---|---|---|---|---|---|
| Synth 1, 4 | | Me, Me | | | | | I |
| Synth 3 | | Me, H | | | | | Br |
| Synth 3 | | Me, | | | | | I |
| Synth 6 | | H, H | | | | | Br |
| Synth 10 | | $2R^1$ = cyclo butyl | | | | | I |
| 11 | | Me, Et | | | | | I |
| 16 | | Me, Et | | | | | Br |
| 22 | 21 | | Me | CN | Me | | I |
| 24 | 21 | | | CN | Me | | Br |
| 28 | 21 | | | CN | Me | | I |
| 40 | 21 | | Et | CN | Me | | Br |
| 44 | 21 | | | CN | Me | | I |
| 45 | 21 | | n-Pr | CN | Me | | I |
| 53 | 21 | | n-Pr | CN | Me | | I |

(continued)

| Compound Number | Nitrile | $R_1$ | $R_2$ | $R_3$ | $R_4$ | $R_5$ | X |
|---|---|---|---|---|---|---|---|
| Synth 60 | | Me, H | | | | | Br |
| 60 | | Me, | | | | | I |
| Synth 63 | | Me | | | | | Br |
| Synth 71, 73, 74, 77, 78 | | | Me | COOMe | Me | | I |
| 82 | | | i-Pr | CN | H | | I |
| 100 | | | | | | Me | Br |
| 102 | | | | | | Me | Br |
| 108 | | | | | | Me | Br |
| 109 | | | | | | Me | Br |
| 126 | | | Et | COOMe | Me | | Br |
| 130 | | | n-Pr | COOMe | Me | | I |
| 133 | | | | COOMe | Me | | Br |
| 136 | | | i-Pr | COOMe | Me | | I |
| 137 | | | Allyl | COOMe | Me | | I |
| Synth 140 | | Me, Me | | | | | Br |
| 143 | 21 | | | CN | Me | | Br |

A representative procedure is given for the synthesis of Table 1 Molecule 22.
A solution of n-butyllithium (2.2M in cyclohexane - 1 eq.) was added drop wise to an ice cold solution of diisopropylamine (1 eq.) in dry THF (0.5 M). After stirring for 10 minutes at this temperature, 3,4-dimethylcyclohex-3-ene-1-carbonitrile (1 eq.) was added to the mixture. Prenyl bromide (1 eq.) was added after another mixing of 10 minutes at 0 °C. Reaction conversion was followed by GC-MS and seen as complete after 15 minutes stirring at 0 °C. The reaction was quenched by addition of a saturated $NH_4Cl$ aqueous solution and extracted with $Et_2O$. The combined organic layers were dried over $MgSO_4$ and concentrated under reduced pressure. The resulting oil was purified using a quick filtration over silica by elution with a petroleum ether- $Et_2O$ mixture (9-1). Concentration of the eluent under reduced pressure resulted in the compound as a colorless oil (96% yield).

Synthesis of Table 1 Molecule Number 1, 3, 10, 12, 13, 17, 19, 23, 26, 27, 41, 42, 46, 48, 52, 54, 57, 58, 59, 96, 110, 119, 125, 138, 140 and 144 or intermediates used for synthesis of Table 1 Molecule Number 4 and 63:

NC, $R^3$, $R^1$, $R^2$ — i. $R^4Li$; ii. $H_2SO_4$; THF — O, $R^4$, $R^3$, $R^1$, $R^2$

i. $R^4Li$
ii. $H_2SO_4$
THF

i. 2.2 $R^4Li$
ii. $H_2SO_4$
THF

| Compound Number | Nitrile | $R^1$ | $R^2$ | $R^3$ | $R^4$ | $R^5$ | $R^6$ |
|---|---|---|---|---|---|---|---|
| 1 | | Me | Me | | n-Bu | | |
| 3 | | Me | i-Pr | | Me | | |
| Synth 4 | | Me | Me | | Me | | |
| 10 | | $R^1R^2$ = cyclobutyl | | | Et | | |
| 12 | 11 | Me | Et | | Me | | |
| 13 | 11 | Me | Et | | n-Bu | | |
| 17 | | Me | Et | | Me | | |
| 19 | | Me | Et | | n-Bu | | |
| 23 | 22 | | | | Me | Me | |
| 26 | 24 | | | | Me | | |
| 27 | 22 | | | | n-Bu | Me | |
| 41 | 40 | | | | Me | Et | |
| 42 | 40 | | | | n-Bu | Et | |
| 46 | 44 | | | | Me | | |
| 48 | 45 | | | | Me | n-Pr | |
| 52 | 28 | | | | Me | | |
| 54 | 22 | | | | i-Bu | Me | |
| 57 | 22 | | | | Et | Me | |
| 58 | 40 | | | | i-Bu | Et | |
| 59 | 40 | | | | Et | Et | |
| Synth 63 | | Me | Me | | Me | | |
| 96 | | | | | Me | | |

(continued)

| Compound Number | Nitrile | R1 | R2 | R3 | R4 | R5 | R6 |
|---|---|---|---|---|---|---|---|
| 110 | | | | | Me | | |
| 119 | 60 | Me | Allyl | | Me | | |
| 125 | | | | | Me | | |
| 138 | | | | | Me | | |
| 140 | | Me | Me | | Me | | |
| 144 | 143 | | | | Me | n-Bu | |

A representative procedure is given for the synthesis of Table 1 Molecule 23. A methyllithium solution (1.2 equiv.) was added drop wise to a solution of the nitrile 1,3,4-trimethylcyclohex-3-ene-1-carbonitrile (1 eq.) in dry THF (0.5M) at -20 °C. After stirring for 15 minutes at -10 / -20 °C, full conversion was observed by GC-MS. The reaction was quenched with a $H_2SO_4$ solution (2M - 2 eq.) and stirred at ambient temperature till full hydrolysis of the in situ formed imine was observed. The mixture was then extracted with $Et_2O$ and washed with a saturated $NaHCO_3$ aqueous solution. The combined organic phases were dried over $MgSO_4$ and concentrated under reduced pressure. The resulting oil was purified using a quick filtration over silica gel by eluting with a petroleum ether - $Et_2O$ mixture (9-1). Concentration of the eluent under reduced pressure resulted in the compound as a colorless oil (95% yield).

Synthesis of Table 1 Molecule Number 6, 14, 15, 18, 20, 25, 43, 47, 49, 56, 63, 86, 89, 101, 111, 112, 115, 120, 124, 128, 129, 131, 132, 134, 135, 141 and 145 or intermediates used for synthesis of Table 1 Molecule Number 68, 69, 72, 73, 74, 75, 76, 77, 78, 79, 104 & 111:

LiAlH4 / Et2O

LiAlH4 / Et2O

LiAlH4 / Et2O

| Compound Number | Carbonyl Compound | R1 | R2 | R3 | R4 | R5 | R6 | R7 | R8 | R9 | R10 |
|---|---|---|---|---|---|---|---|---|---|---|---|
| 6 | | H | H | | H | H | | | | | |
| 14 | 12 | Me | Et | | Me | ME | | | | | |
| 15 | 13 | Me | Et | | n-Bu | n- Bu | | | | | |
| 18 | 17 | Me | Et | | Me | Me | | | | | |
| 20 | 19 | Me | Et | | n-Bu | n- Bu | | | | | |
| 25 | 23 | | | | | | Me | Me | H | Me | Me |
| 3 | 42 | | | | | | Me | n-Bu | H | Me | n-Bu |
| 47 | 46 | | | | | | | Me | H | Me | Me |
| 49 | 48 | | | | | | n-Pr | Me | H | Me | Me |
| 56 | 26 | | | | | | | Me | H | Me | Me |
| 63 | | Me | Me | | Me | Me | | | | | |
| Synth 68 | | | | | | | Me | H | H | Me | H |
| Synth 69 | | | | | | | n-Pr | Me | H | Me | Me |
| Synth 72, 75, 76, 79 | | | | | | | H | OMe | H | Me | H |
| Synth 73, 74, 77, 78 | | | | | | | Me | OMe | H | Me | H |
| 86 | | | | | | | Me | Me | Me | Me | Me |
| 89 | 3rd equation | | | | | | | | | | |
| 101 | 100 | Me | Me | | OMe | H | | | | | |
| Synth 104, 111 | | Me | Me | | OMe | H | | | | | |

(continued)

| Compound Number | Carbonyl Compound | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|
| 112 | 109 | Me | Me | | OMe | H | | | | | |
| 115 | 108 | Me | Me | | OMe | H | | | | | |
| 120 | 119 | Me | Allyl | | Me | Me | | | | | |
| 124 | 123 | | | | | | H | OMe | H | Me | H |
| 127 | 126 | | | | | | Et | OMe | H | Me | H |
| 128 | 41 | | | | | | Et | Me | H | Me | Me |
| 129 | 136 | | | | | | i-Pr | OMe | H | Me | H |
| 131 | 122 | | | | | | Me | OMe | H | Me | H |
| 132 | 130 | | | | | | n-Pr | OMe | H | Me | H |
| 134 | 133 | | | | | | | OMe | H | Me | H |
| 135 | 137 | | | | | | Allyl | OMe | H | Me | H |
| 141 | 140 | Me | Me | | Me | Me | | | | | |
| 145 | 144 | | | | | | n-Bu | Me | H | Me | Me |

A representative procedure is given for the synthesis of Table 1 Molecule **25.**
To a solution of ketone 1-(1,3,4-trimethylcyclohex-3-en-1-yl)ethan-1-one (1 eq.) in dry THF (0.5 M) was added portion wise lithium-aluminiumhydride (0.5 eq.) at 0 °C. Reaction completion was observed by GC-MS after 15 minutes of stirring at ambient temperature. The mixture was cooled to 0 °C and consequently was added: water (same amount of mL as mg hydride used), 15% NaOH solution (same amount of mL as mg hydride used) & water (2 times amount of mL as mg hydride used). This quenching was followed by stirring for 1 hour at ambient temperature. The resulting mixture was filtered over celite and the filter was washed with $Et_2O$. Concentration of the filtrate under reduced pressure resulted in the compound as a colorless oil (98% yield).

Synthesis of Table 1 Molecule Number 6, 50, 51 & 55 or intermediates used for synthesis of Table 1 Molecule Number 61 and 121:

DIBAL, $CH_2Cl_2$

DIBAL

| Compound Number | Nitrile | $R^1$ | $R^2$ | $R^3$ | $R^4$ | $R^5$ |
|---|---|---|---|---|---|---|
| 6 | | H | H | | | |
| 50 | 45 | | | | Me | n-Pr |
| 51 | 44 | | | | Me | |
| 55 | 24 | | | | Me | |
| Synth 61 | | Me | H | | | |
| Synth 121 | 21 | | | | Me | H |

A representative procedure is given for the synthesis of Table 1 Molecule **50.**
To a solution of compound 45 (1 eq.) in dry $CH_2Cl_2$ (0.5 M) at -40 °C was added drop wise a diisobutylaluminiumhydride (1.3 eq.) solution (1.1 M cyclohexane). The resulting mixture was allowed to warm to ambient temperature for 2 hours. Reaction completion was observed by GC-MS. The mixture was cooled to 0 °C and a saturated aqueous sodium potassium tartrate solution was added carefully. This quenching was followed by stirring for 2 hours at ambient temperature. The resulting mixture was extracted with $CH_2Cl_2$, dried over $MgSO_4$ and reduced under reduced pressure to yield the pure aldehyde (94% yield).

Synthesis of carboxylic acids used for the synthesis of Table 1 Molecules 8:

KHMDS
iso-butyraldehyde
THF

To a solution of the phosphonium bromide [50889-29-7] (1.2 eq.) in dry THF (0.3 M) was added KHMDS (Potassiumhexamethyldisilazane - 2.4 eq.) at ambient temperature. After stirring for 30 minutes at the same temperature, a solution of iso-butyraldehyde (1 eq.) in dry THF (0.3 M) was added drop wise. The mixture was stirred for 3 hours, quenched with water (0.3 M) and extracted with $Et_2O$. The resulting water layer was acidified with a HCl aqueous solution (10%) till pH 2 was obtained. This mixture was extracted with $Et_2O$ and the extracts were dried over $MgSO_4$ and concentrated under reduced pressure. The resulting oil was purified using a quick filtration over silica gel by eluting with a petroleum ether - $Et_2O$ mixture (1-1). Concentration under reduced pressure resulted in the compound as a slightly yellow oil. (89% yield)

Synthesis of Table 1 Molecule Number 8:

catalyst
MeOH

A solution of the carboxylic acid (1 eq.), methanol (2 eq.) and the Ishihara catalyst dimesitylammonium pentafluorobenzenesulfonate [850629-65-1] (1 mol%) in heptane (0.5 M) was heated at 60°C. Reaction completion was observed by GC-MS after 3 hours stirring.
The mixture was then washed with an aqueous HCl solution (1 M). The organic phase was dried over $MgSO_4$ and concentrated under reduced pressure. The resulting oil was purified using a quick filtration over silica gel by eluting with a petroleum ether - $Et_2O$ mixture (9-1). Concentration of the eluent under reduced pressure resulted in the compound as a colorless oil (91% yield).

**Synthesis of Table 1 Molecule Number 61:**

$CH_2O$, $K_2CO_3$

Acetone, pTSA

Step 1. To a solution of the aldehyde (1 eq.) in ethanol (0.5 M) was added formaldehyde (3.5 eq., 40% aqueous solution) and potassium carbonate (0.6 eq., 30% aqueous solution). The resulting mixture was heated to reflux and stirred overnight. The reaction was diluted with diethyl ether, dried over $MgSO_4$ and concentrated under reduced pressure (95% yield).
Step 2. The resulting diol was dissolved in acetone (0.5 M) and 2,2-dimethoxypropane (5 eq.) and pTSA (2%) were added. After stirring for 2 hours at 45 °C, full conversion has been observed. The reaction was diluted with diethyl ether, washed with a sodium bicarbonate solution, dried over $MgSO_4$ and concentrated under reduced pressure (77% yield).

Synthesis of Table 1 Molecule Number 121:

Step 1. To a solution of the aldehyde (1 eq.) in ethanol (0.5 M) was added formaldehyde (3.5 eq., 40% aqueous solution) and potassium carbonate (0.6 eq., 30% aqueous solution). The resulting mixture was heated to reflux and stirred overnight. The reaction was diluted with diethyl ether, dried over $MgSO_4$ and concentrated under reduced pressure (Quant.).

Step 2. The resulting diol was dissolved in acetone (0.5 M) and 2,2-dimethoxypropane (5 eq.) and pTSA (2%) were added. After stirring for 2 hours at 45 °C, full conversion has been observed. The reaction was diluted with diethyl ether, washed with a sodium bicarbonate solution, dried over $MgSO_4$ and concentrated under reduced pressure (77% yield).

Synthesis of Table 1 Molecule Number 21, 64, 66, 105, 113, 116, 122, 123, 146 or intermediates used for synthesis of Table 1 Molecule Number 62:

| Compound Number | $R_1$ | $R_2$ |
|---|---|---|
| 21 | CN | H |
| 64 | $CO_2$n-Pr | H |
| 66 | $CO_2$n-Bu | H |
| 105 | $CO_2$Et | H |
| 113 | $CO_2$i-Bu | H |
| 116 | $CO_2$t-Bu | H |
| 122 | $CO_2$Me | Me |
| 123 | $CO_2$Me | H |
| 146 | $CO_2$i-Pr | H |

A representative procedure is given for the synthesis of Table 1 Molecule **21.**

A solution of acrylonitrile (1 eq.) and 2,3-dimethyl-1,3-butadiene (1 eq.) in toluene (2 M) was pumped through a tubular (SS, ID 1.0 mm) flow reactor (10 mL) at 220 °C with a residence time of 15 minutes. The reacted mixture was concentrated under reduced pressure which resulted in the desired cyclic nitrile (83% yield).

Synthesis of Table 1 Molecule Number 62, 65, 67, 68, 69, 70, 71, 76, 87, 88, 91, 97, 106, 114 & 117:

$R_1$, $R_2$, $R_3$, $R_4$, $R_4$ — Pd/C, $H_2$ → $R_1$, $R_2$, $R_3$, $R_4$, $R_4$

| Compound Number | $R_1$ | $R_2$ | $R_3$ | $R_4$ |
|---|---|---|---|---|
| 62 | | H | H | Me |
| 65 | | H | H | Me |
| 67 | | H | H | Me |
| 68 | $CH_2OH$ | Me | H | Me |
| 69 | HO | n-Pr | H | Me |
| 70 | $CO_2Me$ | H | H | Me |
| 71 | $CO_2Me$ | Me | H | Me |
| 76 | $CH_2OH$ | H | H | Me |
| 87 | | Me | Me | Me |
| 88 | HO | Me | Me | Me |
| 91 | | Me | Me | H |
| 97 | HO | Me | Me | H |
| 106 | | H | H | Me |
| 114 | | H | H | Me |
| 117 | | H | H | Me |

A representative procedure is given for the synthesis of Table 1 Molecule **68.**
A mixture of the alkene (1,3,4-trimethylcyclohex-3-en-1-yl)methanol (1 eq.) and palladium on carbon (5%) in EtOAc (0.3 M) was stirred under hydrogen gas atmosphere for 3 hours at ambient temperature. The reaction mixture was filtered and concentrated under reduced pressure. The resulting oil was purified using a quick filtration over silica gel by eluting with a petroleum ether - $Et_2O$ mixture (9-1). Concentration of the eluent under reduced pressure resulted in the compound as an orange oil (98% yield).

Synthesis of Table 1 Molecule Number 72, 73, 78, 79, 80, 81, 92, 99, 107 & 111:

| Compound Number | $R_1$ | $R_2$ | $R_3$ | $R_4$ | $R_5$ | $R_6$ |
|---|---|---|---|---|---|---|
| 72 | H | H | H | Me | | |
| 73 | H | Me | H | Me | | |
| 78 | H | Me | H | | Me | |
| 79 | H | H | H | | Me | |
| 80 | Me | Me | H | Me | | |
| 81 | Me | Me | H | | Me | |
| 92 | 3rd equation | | | | | |
| 99 | Me | Me | Me | H | | |
| 107 | | | | | | |
| 111 | | | | | | |

A representative procedure is given for the synthesis of Table 1 Molecule **73.**

A mixture of the alcohol (1,3,4-trimethylcyclohex-3-en-1-yl)methanol (1 eq.), acetic acid anhydride (1 eq.) and potassium carbonate (catalytic) in EtOAc (0.5 M) was heated to reflux. Full conversion was obtained after 24 hours. The reaction mixture was diluted with $Et_2O$ and washed with aqueous sodium bicarbonate solution. The organic layer was dried over $MgSO_4$ and concentrated under reduced pressure. The resulting oil was purified using a quick filtration over silica gel by eluting with a petroleum ether - $Et_2O$ mixture (9-1). Concentration of the eluent under reduced pressure resulted in the compound as an orange oil (91% yield).

Synthesis of Table 1 Molecule Number 74, 75, 77, 90, 94, 95, 103 & 104:

| Compound Number | $R_1$ | $R_2$ | $R_3$ | $R_4$ | $R_5$ |
|---|---|---|---|---|---|
| 74 | H | Me | H | Me | |
| 75 | H | H | H | Me | |
| 77 | H | Me | H | Me | |
| 90 | 2nd equation | | | | |
| 94 | Me | Me | Me | Me | |
| 95 | Me | Me | Me | H | |
| 103 | | | | | |
| 104 | | | | | |

A representative procedure is given for the synthesis of Table 1 Molecule **75.**
A mixture of the alcohol (3,4-dimethylcyclohex-3-en-1-yl)methanol (1 eq.), formic acid (20 eq.) and iodine (0.25 mol%) was stirred overnight. The reaction mixture was quenched by the addition of an aqueous $Na_2S_2O_3$ solution and 10% aqueous NaOH solution. The resulting mixture was extracted with $Et_2O$ and washed with an aqueous sodium bicarbonate solution. The organic layer was dried over $MgSO_4$ and concentrated under reduced pressure. The resulting oil was purified using a quick filtration over silica gel by eluting with a petroleum ether - $Et_2O$ mixture (9-1). Concentration of the eluent under reduced pressure resulted in the compound as an orange oil (76% yield).

Synthesis of Table 1 Molecule Number 84, 85 & 118:

| Compound Number | $R_1$ | $R_2$ | $R_3$ | $R_4$ |
|---|---|---|---|---|
| 84 | Me | Me | Me | Me |
| 85 | Me | Me | Me | H |

(continued)

| Compound Number | $R_1$ | $R_2$ | $R_3$ | $R_4$ |
|---|---|---|---|---|
| 118 | H | H | Ph | Me |

A representative procedure is given for the synthesis of Table 1 Molecule **84.** To a solution of $BF_3.Et_2O$ (0.1 eq.) in toluene (3 M) was added 3-methylpent-3-en-2-one (1.2 eq.) dissolved in toluene (8 M) at -5 °C. After stirring for 20 minutes, a solution of diene (1.0 eq.) in toluene (3 M) was added at -5 °C. The resulting mixture was heated to 50 °C and stirred overnight. The reaction was quenched by the addition of 30% NaOH solution and extracted with $Et_2O$. The organic layer was dried over $MgSO_4$ and concentrated under reduced pressure. The resulting oil was purified using a quick filtration over silica gel by eluting with a petroleum ether - $Et_2O$ mixture (9-1). Concentration of the eluent under reduced pressure resulted in the compound as an orange oil (79% yield).

Synthesis of Table 1 Molecule Number 2, 5 & 7:

nBuLi, RX

THF

| Compound Number | R | X |
|---|---|---|
| 2 | | I |
| 5 | | Cl |
| 7 | | Br |

A representative procedure is given for the synthesis of Table 1 Molecule **2.** A solution of 2-methyl-furan (2 eq.) in THF (0.5 M) was added drop wise a n-butyllithium solution (2 eq., 2.2 M) at 0 °C. After stirring for 20 minutes, the halide 5-iodo-2-methylpent-2-ene (1 eq.) was added to the mixture. Full conversion has been observed after stirring for 1 hour at ambient temperature. The reaction was quenched by addition of a saturated $NH_4Cl$ aqueous solution and extracted with $Et_2O$. The combined organic layers were dried over $MgSO_4$ and concentrated under reduced pressure. The resulting oil was purified using a quick filtration over silica by elution with a petroleum ether- $Et_2O$ mixture (9-1). Concentration of the eluent under reduced pressure resulted in the compound as a colorless oil (88% yield).

Synthesis of Table 1 Molecule Number 98:

OH

$Cu(OTf)_2$

O +

O

A mixture of 1-(1,3,4,6-tetramethylcyclohex-3-en-1-yl)ethan-1-ol (1 eq.) and $Cu(OTf)_2$ (3 mol%) in toluene is stirred overnight at 65 °C. The resulting mixture is concentrated under reduced pressure and purified using a quick filtration over silica by elution with a petroleum ether- $Et_2O$ mixture (9-1). Concentration of the eluent under reduced

pressure resulted in the compound as a colorless oil (90% yield).

Synthesis of Table 1 Molecule Number 4:

NH$_2$OH.HCl
NaOAc

A mixture of ketone 3,3,7-trimethyloct-6-en-2-one (1 eq.), NH$_2$OH.HCl (1.5 eq.) and NaOAc (3 eq.) in ethanol (0.5 M) was stirred for 3 hours at ambient temperature. The reaction was quenched by addition of a saturated NH$_4$Cl aqueous solution and extracted with Et$_2$O. The combined organic layers were dried over MgSO$_4$ and concentrated under reduced pressure. The resulting oil was purified using a quick filtration over silica by elution with a petroleum ether- Et$_2$O mixture (1-1). Concentration of the eluent under reduced pressure resulted in the compound as a colorless oil (97% yield).

Synthesis of Table 1 Molecule Number 83:

NaHCO$_3$, Br$_2$, NaOMe

To a mixture of Pulegone (1 eq.) and sodium bicarbonate (0.3 eq.) in Et$_2$O (1 M) was added bromine (1.5 eq.) over 30 minutes at 0 °C. The resulting mixture has been filtered and NaOMe has been added to this solution at 0 °C. Full conversion has been observed after 1 hour stirring. The resulting reaction mixture has been quenched by the addition of 5% HCl solution until pH 7. After stirring for 2 more hours, the mixture has been extracted by Et$_2$O and washed with water. The organic layer was dried over MgSO$_4$ and concentrated under reduced pressure. The resulting oil was purified using a quick filtration over silica gel by eluting with a petroleum ether - Et$_2$O mixture (8-2). Concentration of the eluent under reduced pressure resulted in the compound as an orange oil (80% yield).

Synthesis of 3-ethoxy-4-hydroxybenzonitrile used in the synthesis of Table 1 Molecule Number 31, 32, 34, 35, 36 & 37:

NH$_2$OH.HCl
CH$_3$CN

A solution of ethyl vanillin (1 eq., 18 mmol) and hydroxylamine hydrochloride (1.2 eq.) in acetonitrile (1 M) was stirred for 1h30 at reflux temperature. The reaction was quenched with water and extracted with diethyl ether. The organic layers were washed with a saturated aqueous NaHCO$_3$ solution, dried over MgSO$_4$ and concentrated under reduced pressure. Recrystallization from di-*iso*-propyl ether delivered the pure nitrile as white crystals (85% yield).

Synthesis of Table 1 Molecule Number 31, 32, 34, 35, 36 & 37:

| Compound Number | R |
|---|---|
| 31 | Et |
| 32 | n-Bu |
| 34 | i-Bu |
| 35 | s-Bu |
| 36 | t-Bu |
| 37 | i-Pr |

A representative procedure is given for the synthesis of Table 1 Molecule **32.**
A n-butyllithium solution (2.1 equiv.) was added drop wise to a solution of the nitrile 3-ethoxy-4-hydroxybenzonitrile (1 eq.) in dry THF (0.5M) at -20 °C. After stirring for 1 hour at ambient temperature, full conversion was observed by GC-MS. The reaction was quenched with a $H_2SO_4$ solution (2M - 2 eq.) and stirred at ambient temperature till full hydrolysis of the in situ formed imine was observed (2 hours). The mixture was then extracted with $Et_2O$ and washed with a saturated $NaHCO_3$ aqueous solution. The combined organic phases were dried over $MgSO_4$ and concentrated under reduced pressure. The resulting product was purified using a quick filtration over silica gel by eluting with a petroleum ether - $Et_2O$ mixture (1-1). Concentration of the eluent under reduced pressure resulted in the compound as white crystals (98% yield).

Synthesis of Table 1 Molecule Number 29, 30 & 39:

| Compound Number | R |
|---|---|
| 29 | n-Pr |
| 30 | i-Pr |
| 39 | i-Bu |

A representative procedure is given for the synthesis of Table 1 Molecule **29.**
To a solution of iso-vanillin (1 eq.) in MEK (0.5 M) was added n-PrI (1.1 eq.), potassium carbonate (3 eq.) and potassium iodide (1 %). The resulting mixture was heated to reflux temperature and stirred overnight. The reaction was concentrated under reduced pressure, quenched by addition of a saturated $NH_4Cl$ aqueous solution and extracted with $Et_2O$. The combined organic layers were dried over $MgSO_4$ and concentrated under reduced pressure. The resulting oil was purified using a quick filtration over silica by elution with a petroleum ether- $Et_2O$ mixture (9-1). Concentration of the eluent under reduced pressure resulted in the compound as a colorless oil (99% yield).

Synthesis of Table 1 Molecule Nos. 33 & 38:

| Compound Number | R |
|---|---|
| 33 | n-Pr |
| 38 | [cyclopropylmethyl structure] |

A representative procedure is given for the synthesis of Table 1 Molecule No. 33.

A mixture of *n*-PrOH (1.3 M - 12 mL) and sodium metal (4 eq.) was heated at 110 °C till all sodium was dissolved. DMF (1.3 M - 12 mL) was added to this mixture at ambient temperature and degassed under reduced pressure. CuCl (1.45 equiv.) was added and stirred for 10 minutes at the same temperature. After adding the bromide (1 equiv.), the mixture was heated to 120 °C. Full conversion was observed after 30 minutes by GC-MS analysis. The resulting reaction mixture was acidified with an aqueous solution of HCl (2 M),extracted with EtOAc, washed with water and dried over $MgSO_4$. Concentration under reduced pressure resulted in a brown oil which was purified by column chromatography (eluent: petroleum ether/EtOAc - 8/2). The product was isolated as a yellow oil (80% yield).

Synthesis of Table 1 Molecule No. 9:

Step 1. A mixture of the epoxide table 1 molecule number (1 equiv. - 19 mmol), NaI (3 equiv.), $CeCl_3.7H_20$ (1.2 equiv.) in acetonitrile (0.5 M) was stirred at 40°C for 2 hours. Full conversion was observed by GC-MS. The reaction was quenched by the addition of water and extracted with $Et_2O$. The organic phase was washed with an aqueous $Na_2S_2O_3$ solution, dried over $MgSO_4$ and concentrated under reduced pressure. This yielded the pure product (70% yield).

Step 2. A catalytic amount of DMAP (0.05 equiv.) was added to a mixture of the iodohydrine (1 equiv. - 10 mmol), pyridine (1.5 equiv.) and acetic anhydride (2 equiv.) in $CH_2Cl_2$ (0.5 M) at ambient temperature. Full conversion was observed after 1 hour by GC-MS analysis. The reaction was quenched by the addition of water and extracted with $Et_2O$. The organic phase was washed with an aqueous 1M HCl solution, dried over $MgSO_4$ and concentrated under reduced pressure. This yielded the pure product (95% yield).

Step 3. A solution of the iodide (1 equiv. - 15 mmol) and DBU (2 equiv.) in toluene (0.5 M) was stirred for 2 hours

at 70 °C. Full conversion was observed by GC-MS analysis. The reaction was concentrated under reduced pressure and retaken in $Et_2O$, washed with an aqueous 1M HCl solution, dried over $MgSO_4$ and again concentrated under reduced pressure. This resulted in pure allylic acetate (96% yield).

Synthesis of Table 1 Molecule Number 139:

Step 1. To a solution of the carboxylic acid (1 eq.) in dichloromethane (0.5 M) was added carbonyldiimidazole (1 eq.). After 10 minutes of stirring at room temperature N,O-dimethylhydroxylamine hydrochloride (1.2 eq.) was added. The resulting mixture was stirred overnight at room temperature. The reaction was diluted with water, washed with a HCl solution (1 M), washed with a sodium bicarbonate solution, dried over $MgSO_4$ and concentrated under reduced pressure (72% yield).

Step 2. The resulting Weinreb amide (1 eq.) was added to a cooled (-20 ° C) solution of freshly prepared lithium diisopropylamine (2 eq.) in tetrahydrofuran. After stirring for 40 minutes, full conversion has been observed. A solution of ammonium chloride was added and the mixture was extracted with ethyl acetate, dried over MgSO4 and concentrated under reduced pressure. Further purification by column chromatography (eluent: petroleum ether/ether - 5/5) yielded a yellowish oil. (50% yield).

Synthesis of Table 1 Molecule Number 142:

CF3SO3H

To a solution of the alcohol (1eq.) in dichloromethane (0.25M) was added trifluoromethanesulfonic acid (2 drops). The resulting mixture was stirred overnight at room temperature. The reaction mixture was diluted with dichloromethane, washed with a NaHCO3 solution, washed with water, dried over MgSO4 and concentrated under reduced pressure. Further purification by column chromatography (eluent: petroleum ether/ether - 9/1) yielded a yellowish oil. (86% yield).

Example 2: Preformed Amine Reaction Product

The following ingredients are weighted off in a glass vial:

1. 50% of the perfume material comprising one or more Table 1 PRMs
2. 50% of Lupasol WF (CAS# 09002-98-6) from BASF, is put at 60°C in warm water bath for 1 hour before use.

Mixing of the two ingredients is done by using the Ultra-Turrax T25 Basic equipment (from IKA) during 5 minutes. When the mixing is finished the sample is put in a warm water bath at 60°C for ± 12 hours. A homogenous, viscous material is obtained.

In the same way as described above different ratios between the components can be used:

| Weight % | | | | | |
|---|---|---|---|---|---|
| Perfume Material | 40 | 50 | 60 | 70 | 80 |
| Lupasol WF | 60 | 50 | 40 | 30 | 20 |

Example 3: 84wt% Core / 16wt% Wall Melamine Formaldehyde (MF) Capsule (PAD Reservoir System

17 grams of butyl acrylate-acrylic acid copolymer emulsifier (Colloid C351, 25% solids, pka 4.5-4.7, (Kemira Chemicals, Inc. Kennesaw, Ga. U.S.A.) and 17 grams of polyacrylic acid (35% solids, pKa 1.5-2.5, Aldrich) are dissolved and mixed in 200 grams deionized water. The pH of the solution is adjusted to pH of 6.0with sodium hydroxide solution. 7grams of partially methylated methylol melamine resin (Cymel 385, 80% solids, (Cytec Industries West Paterson, N.J., U.S.A.)) is added to the emulsifier solution. 200 grams of perfume oil is added to the previous mixture under mechanical agitation and the temperature is raised to 45° C. After mixing at higher speed until a stable emulsion is obtained, the second solution and 4 grams of sodium sulfate salt are added to the emulsion. This second solution contains 3 grams of polyacrylic acid polymer (Colloid C121, 25% solids (Kemira Chemicals, Inc. Kennesaw, Ga. U.S.A.), 100 grams of distilled water, sodium hydroxide solution to adjust pH to 6.0, 10 grams of partially methylated methyol melamine resin (Cymel 385, 80% Cytec). This mixture is heated till 85C and maintained 8 hours with continuous stirring to complete the encapsulation process. 23 grams of acetoacetamide (Sigma-Aldrich, Saint Louis, Mo. U.S.A.) is added to the suspension. Salts and structuring agents can then still be added to the slurry.

Example 4: Process of Making a Polymer Assisted Delivery (PAD) Matrix System

A mixture comprising 50% of a perfume composition comprising one or more Table 1 PRMs, 40% of carboxyl-terminated Hypro™ RLP 1300X18 (CAS#0068891-50-9) from nanoresins, (put at 60°C in warm water bath for 1 hour before mixing) and 10% of Lupasol® WF(CAS# 09002-98-6) from BASF (put at 60°C in warm water bath for 1 hour before mixing). Mixing is achieved by mixing for five minutes using a Ultra-Turrax T25 Basic equipment (from IKA). After mixing, the mixture is put in a warm water bath at 60°C for ± 12 hours. A homogenous, viscous and sticky material is obtained.

In the same way as described above different ratios between the components can be used:

| Weight % | | | | | |
|---|---|---|---|---|---|
| Perfume composition | 40 | 50 | 60 | 70 | 80 |
| Lupasol® WF | 12 | 10 | 8 | 6 | 4 |
| Hypro™ RLP CTBN1300X18 | 48 | 40 | 32 | 24 | 16 |

| Weight % | | | | | | | | |
|---|---|---|---|---|---|---|---|---|
| Perfume composition | 50 | 50 | 50 | 50 | 50 | 50 | 50 | 50 |
| Lupasol® WF | 2.5 | 5 | 7.5 | 10 | 12.5 | 15 | 17.5 | 20 |
| Hypro™ RLP 1300X18 | 47.5 | 45 | 42.5 | 40 | 37.5 | 35 | 32.5 | 30 |

Examples 5-52: Product Formulation

Non-limiting examples of product formulations containing PRMs disclosed in the present specification perfume and amines summarized in the following table.

Examples 5 - 10

Granular laundry detergent compositions for hand washing or washing machines, typically top-loading washing machines.

| | 5 (wt %) | 6 (wt %) | 7 (wt %) | 8 (wt %) | 9 (wt %) | 10 (wt %) |
|---|---|---|---|---|---|---|
| Linear alkylbenzenesulfonate | 20 | 22 | 20 | 15 | 19.5 | 20 |
| $C_{12-14}$ Dimethylhydroxyethyl ammonium chloride | 0.7 | 0.2 | 1 | 0.6 | 0.0 | 0 |
| AE3S | 0.9 | 1 | 0.9 | 0.0 | 0.4 | 0.9 |
| AE7 | 0.0 | 0.0 | 0.0 | 1 | 0.1 | 3 |
| Sodium tripolyphosphate | 5 | 0.0 | 4 | 9 | 2 | 0.0 |
| Zeolite A | 0.0 | 1 | 0.0 | 1 | 4 | 1 |
| 1.6R Silicate ($SiO_2$:$Na_2O$ at ratio 1.6:1) | 7 | 5 | 2 | 3 | 3 | 5 |
| Sodium carbonate | 25 | 20 | 25 | 17 | 18 | 19 |
| Polyacrylate MW 4500 | 1 | 0.6 | 1 | 1 | 1.5 | 1 |
| Random graft copolymer[1] | 0.1 | 0.2 | 0.0 | 0.0 | 0.05 | 0.0 |
| Carboxymethyl cellulose | 1 | 0.3 | 1 | 1 | 1 | 1 |
| Stainzyme® (20 mg active/g) | 0.1 | 0.2 | 0.1 | 0.2 | 0.1 | 0.1 |
| Protease (Savinase®, 32.89 mg active/g) | 0.1 | 0.1 | 0.1 | 0.1 | | 0.1 |
| Amylase - Natalase® (8.65 mg active/g) | 0.1 | 0.0 | 0.1 | 0.0 | 0.1 | 0.1 |
| Lipase - Lipex® (18 mg active /g) | 0.03 | 0.07 | 0.3 | 0.1 | 0.07 | 0.4 |
| | | | | | | |
| Fluorescent Brightener 1 | 0.06 | 0.0 | 0.06 | 0.18 | 0.06 | 0.06 |
| Fluorescent Brightener 2 | 0.1 | 0.06 | 0.1 | 0.0 | 0.1 | 0.1 |
| DTPA | 0.6 | 0.8 | 0.6 | 0.25 | 0.6 | 0.6 |
| $MgSO_4$ | 1 | 1 | 1 | 0.5 | 1 | 1 |
| Sodium Percarbonate | 0.0 | 5.2 | 0.1 | 0.0 | 0.0 | 0.0 |
| Sodium Perborate Monohvdrate | 4.4 | 0.0 | 3.85 | 2.09 | 0.78 | 3.63 |
| NOBS | 1.9 | 0.0 | 1.66 | 0.0 | 0.33 | 0.75 |
| TAED | 0.58 | 1.2 | 0.51 | 0.0 | 0.015 | 0.28 |
| Sulphonated zinc phthalocyanine | 0.0030 | 0.0 | 0.0012 | 0.0030 | 0.0021 | 0.0 |
| S-ACMC | 0.1 | 0.0 | 0.0 | 0.0 | 0.06 | 0.0 |
| Direct Violet Dye (DV9 or DV99 or DV66) | 0.0 | 0.0 | 0.0003 | 0.0001 | 0.0001 | 0.0 |
| Additional Neat Perfume (2) | 0.5 | 0.5 | 0.5 | 0.5 | 0.5 | 0.5 |
| Amine (1) | 0.1 | 0.5 | 0.0 | 0.01 | 0.02 | 0.00 |
| Perfume Delivery System As Disclosed In The Present Specification Including Examples 2-3 | 0.05 | 0.0 | 0.1 | 0.0 | 0.2 | 0.4 |
| Perfume comprising one or more PRMs from Table 1 | 0.3 | 0.4 | 0.01 | 0.02 | 0.04 | 0.1 |
| Sulfate/Moisture | Balance | | | | | |
| (1) One or more materials comprising an amine moiety as disclosed in the present specification.<br>(2) Optional. | | | | | | |

Examples 11 - 16

Granular laundry detergent compositions typically for front-loading automatic washing machines.

| | 11 (wt %) | 12 (wt%) | 13 (wt%) | 14 (wt%) | 15 (wt%) | 16 (wt%) |
|---|---|---|---|---|---|---|
| Linear alkylbenzenesulfonate | 8 | 7.1 | 7 | 6.5 | 7.5 | 7.5 |
| AE3S | 0 | 4.8 | 1.0 | 5.2 | 4 | 4 |
| C12-14 Alkylsulfate | 1 | 0 | 1 | 0 | 0 | 0 |
| AE7 | 2.2 | 0 | 2.2 | 0 | 0 | 0 |
| $C_{10-12}$ Dimethyl hydroxyethylammonium chloride | 0.75 | 0.94 | 0.98 | 0.98 | 0 | 0 |
| Crystalline layered silicate ($\delta$-$Na_2Si_2O_5$) | 4.1 | 0 | 4.8 | 0 | 0 | 0 |
| Zeolite A | 5 | 0 | 5 | 0 | 2 | 2 |
| Citric Acid | 3 | 5 | 3 | 4 | 2.5 | 3 |
| Sodium Carbonate | 15 | 20 | 14 | 20 | 23 | 23 |
| Silicate 2R ($SiO_2$:$Na_2O$ at ratio 2:1) | 0.08 | 0 | 0.11 | 0 | 0 | 0 |
| Soil release agent | 0.75 | 0.72 | 0.71 | 0.72 | 0 | 0 |
| Acrylic Acid/Maleic Acid Copolymer | 1.1 | 3.7 | 1.0 | 3.7 | 2.6 | 3.8 |
| Carboxymethylcellulo se | 0.15 | 1.4 | 0.2 | 1.4 | 1 | 0.5 |
| Protease - Purafect® (84 mg active/g) | 0.2 | 0.2 | 0.3 | 0.15 | 0.12 | 0.13 |
| Amylase - Stainzyme Plus® (20 mg active/g) | 0.2 | 0.15 | 0.2 | 0.3 | 0.15 | 0.15 |
| Lipase - Lipex® (18.00 mg active/g) | 0.05 | 0.15 | 0.1 | 0 | 0 | 0 |
| Amylase - Natalase® (8.65 mg active/g) | 0.1 | 0.2 | 0 | 0 | 0.15 | 0.15 |
| Cellulase - Celluclean™ (15.6 mg active/g) | 0 | 0 | 0 | 0 | 0.1 | 0.1 |
| TAED | 3.6 | 4.0 | 3.6 | 4.0 | 2.2 | 1.4 |
| Percarbonate | 13 | 13.2 | 13 | 13.2 | 16 | 14 |
| Na salt of Ethylenediamine-N,N'-disuccinic acid, (S,S) isomer (EDDS; | 0.2 | 0.2 | 0.2 | 0.2 | 0.2 | 0.2 |
| Hydroxyethane di phosphonate (HEDP) | 0.2 | 0.2 | 0.2 | 0.2 | 0.2 | 0.2 |
| $MgSO_4$ | 0.42 | 0.42 | 0.42 | 0.42 | 0.4 | 0.4 |
| Perfume | 0.5 | 0.6 | 0.5 | 0.6 | 0.6 | 0.6 |
| Suds suppressor agglomerate | 0.05 | 0.1 | 0.05 | 0.1 | 0.06 | 0.05 |
| Soap | 0.45 | 0.45 | 0.45 | 0.45 | 0 | 0 |
| Sulphonated zinc phthalocyanine (active) | 0.00 07 | 0.0012 | 0.0007 | 0 | 0 | 0 |
| S-ACMC | 0.01 | 0.01 | 0 | 0.01 | 0 | 0 |
| Direct Violet 9 (active) | 0 | 0 | 0.0001 | 0.0001 | 0 | 0 |
| Additional Neat Perfume [(2)] | 0.5 | 0.5 | 0.5 | 0.5 | 0.5 | 0.5 |
| Amine [(1)] | 0.1 | 0.5 | 0.0 | 0.01 | 0.02 | 0.00 |
| Perfume Delivery System As Disclosed In The Present Specification Including Examples 2-3 | 0.05 | 0.0 | 0.1 | 0.0 | 0.2 | 0.4 |
| Perfume comprising one or more PRMs from Table 1 | 0.3 | 0.4 | 0.01 | 0.02 | 0.04 | 0.1 |

(continued)

| | 11 (wt %) | 12 (wt%) | 13 (wt%) | 14 (wt%) | 15 (wt%) | 16 (wt%) |
|---|---|---|---|---|---|---|
| Sulfate/ Water & Miscellaneous | Balance | | | | | |
| (1) One or more materials comprising an amine moiety as disclosed in the present specification.<br>(2) Optional. | | | | | | |

The typical pH is about 10.

Examples 17 - 23 Heavy Duty Liquid laundry detergent compositions

| | 17 (wt%) | 18 (wt%) | 19 (wt%) | 20 (wt%) | 21 (wt%) | 22 (wt%) | 23 (wt%) |
|---|---|---|---|---|---|---|---|
| AES $C_{12-15}$ alkyl ethoxy (1.8) sulfate | 11 | 10 | 4 | 6.32 | 0 | 0 | 0 |
| AE3S | 0 | 0 | 0 | 0 | 2.4 | 0 | 0 |
| Linear alkyl benzene sulfonate/sulfonic acid | 1.4 | 4 | 8 | 3.3 | 5 | 8 | 19 |
| HSAS | 3 | 5.1 | 3 | 0 | 0 | 0 | 0 |
| Sodium formate | 1.6 | 0.09 | 1.2 | 0.04 | 1.6 | 1.2 | 0.2 |
| Sodium hydroxide | 2.3 | 3.8 | 1.7 | 1.9 | 1.7 | 2.5 | 2.3 |
| Monoethano lamine | 1.4 | 1.49 | 1.0 | 0.7 | 0 | 0 | To pH 8.2 |
| Diethylene glycol | 5.5 | 0.0 | 4.1 | 0.0 | 0 | 0 | 0 |
| AE9 | 0.4 | 0.6 | 0.3 | 0.3 | 0 | 0 | 0 |
| AE8 | 0 | 0 | 0 | 0 | 0 | 0 | 20.0 |
| AE7 | 0 | 0 | 0 | 0 | 2.4 | 6 | 0 |
| Chelant (HEDP) | 0.15 | 0.15 | 0.11 | 0.07 | 0.5 | 0.11 | 0.8 |
| Citric Acid | 2.5 | 3.96 | 1.88 | 1.98 | 0.9 | 2.5 | 0.6 |
| $C_{12-14}$ dimethyl Amine Oxide | 0.3 | 0.73 | 0.23 | 0.37 | 0 | 0 | 0 |
| $C_{12-18}$ Fatty Acid | 0.8 | 1.9 | 0.6 | 0.99 | 1.2 | 0 | 15.0 |
| 4-formyl-phenylboronic acid | 0 | 0 | 0 | 0 | 0.05 | 0.02 | 0.01 |
| Borax | 1.43 | 1.5 | 1.1 | 0.75 | 0 | 1.07 | 0 |
| Ethanol | 1.54 | 1.77 | 1.15 | 0.89 | 0 | 3 | 7 |
| A compound having the following general structure: bis(($C_2H_5O$)($C_2H_4O$)n)($CH_3$)-$N^+$-$C_xH_{2x}$-$N^+$-($CH_3$)-bis(($C_2H_5O$)($C_2H_4O$)n), wherein n = from 20 to 30, and x = from 3 to 8, or sulphated or sulphonated variants thereof | 0.1 | 0 | 0 | 0 | 0 | 0 | 2.0 |
| Ethoxylated ($EO_{15}$) tetraethylene pentamine | 0.3 | 0.33 | 0.23 | 0.17 | 0.0 | 0.0 | 0 |
| Ethoxylated Polyethylenimine | 0 | 0 | 0 | 0 | 0 | 0 | 0.8 |
| Ethoxylated hexamethylene diamine | 0.8 | 0.81 | 0.6 | 0.4 | 1 | 1 | |
| 1,2-Propanediol | 0.0 | 6.6 | 0.0 | 3.3 | 0.5 | 2 | 8.0 |
| Fluorescent Brightener | 0.2 | 0.1 | 0.05 | 0.3 | 0.15 | 0.3 | 0.2 |

(continued)

| | 17 (wt%) | 18 (wt%) | 19 (wt%) | 20 (wt%) | 21 (wt%) | 22 (wt%) | 23 (wt%) |
|---|---|---|---|---|---|---|---|
| Hydrogenated castor oil derivative structurant | 0.1 | 0 | 0 | 0 | 0 | 0 | 0.1 |
| Perfume | 1.6 | 1.1 | 1.0 | 0.8 | 0.9 | 1.5 | 1.6 |
| Protease (40.6 mg active/g) | 0.8 | 0.6 | 0.7 | 0.9 | 0.7 | 0.6 | 1.5 |
| Mannanase: Mannaway® (25 mg active/g) | 0.07 | 0.05 | 0.045 | 0.06 | 0.04 | 0.045 | 0.1 |
| Amylase: Stainzyme® (15 mg active/g) | 0.3 | 0 | 0.3 | 0.1 | 0 | 0.4 | 0.1 |
| Amylase: Natalase® (29 mg active/g) | 0 | 0.2 | 0.1 | 0.15 | 0.07 | 0 | 0.1 |
| Xyloglucanase (Whitezyme®, 20mg active/g) | 0.2 | 0.1 | 0 | 0 | 0.05 | 0.05 | 0.2 |
| Lipex® (18 mg active/g) | 0.4 | 0.2 | 0.3 | 0.1 | 0.2 | 0 | 0 |
| Additional Neat Perfume (2) | 0.5 | 0.5 | 0.5 | 0.5 | 0.5 | 0.5 | 0.5 |
| Amine (1) | 0.1 | 0.5 | 0.0 | 0.01 | 0.02 | 0.00 | 0.07 |
| Perfume Delivery System As Disclosed In The Present Specification Including Examples 2-3 | 0.05 | 0.0 | 0.1 | 0.0 | 0.2 | 0.4 | 0.0 |
| Perfume comprising one or more PRMs from Table 1 | 0.7 | 0.5 | 0.8 | 0.05 | 0.6 | 0.1 | 0.6 |
| *Water, dyes & minors | Balance | | | | | | |

* Based on total cleaning and/or treatment composition weight, a total of no more than 12% water
(1) One or more materials comprising an amine moiety as disclosed in the present specification.
(2) Optional.

Examples 24 - 25 Unit Dose Compositions

| Example of Unit Dose detergents | **24** | **25** |
|---|---|---|
| C14 - C15 alkyl poly ethoxylate (8) | 12 | - |
| C12 - C14 alkyl poly ethoxylate (7) | 1 | 14 |
| C12 - C14 alkyl poly ethoxylate (3) sulfate Mono EthanolAmine salt | 8.4 | 9 |
| Linear Alkylbenzene sulfonic acid | 15 | 16 |
| Citric Acid | 0.6 | 0.5 |
| C12-C18 Fatty Acid | 15 | 17 |
| Enzymes | 1.5 | 1.2 |
| PEI 600 EO20 | 4 | - |
| Diethylene triamine penta methylene phosphonic acid or HEDP | 1.3 | - |
| Fluorescent brightener | 0.2 | 0.3 |
| Hydrogenated Castor Oil | 0.2 | 0.2 |
| 1, 2 propanediol | 16 | 12 |
| Glycerol | 6.2 | 8.5 |
| Sodium hydroxide | - | 1 |
| Mono Ethanol Amine | 7.9 | 6.1 |
| Dye | Present | Present |

(continued)

| Example of Unit Dose detergents | **24** | **25** |
|---|---|---|
| PDMS | - | 2.7 |
| Potassium sulphite | 0.2 | 0.2 |
| Additional Neat Perfume (2) | 0.5 | 0.5 |
| Amine (1) | 0.1 | 0.5 |
| Perfume Delivery System As Disclosed In The Present Specification Including Examples 2-3 | 0.05 | 0.0 |
| Perfume comprising one or more PRMs from Table 1 | 0.3 | 0.4 |
| Water | Up to 100p | Up to 100 |
| (1) One or more materials comprising an amine moiety as disclosed in the present specification.<br>(2) Optional. | | |

Example 26 - 31 Bleach & Laundry Additive Detergent Formulations

| Ingredients | 26 | 27 | 28 | 29 | 30 | 31 |
|---|---|---|---|---|---|---|
| AES | 11.3 | 6.0 | 15.4 | 16.0 | 12.0 | 10.0 |
| LAS | 25.6 | 12.0 | 4.6 | - | - | 26.1 |
| MEA-HSAS | - | - | - | 3.5 | - | - |
| DTPA: Diethylene triamine pentaacetic acid | 0.51 | - | 1.5 | - | - | 2.6 |
| 4,5-Dihydroxy-1,3-benzenedisulfonic acid disodium salt | 1.82 | - | - | - | - | 1.4 |
| 1,2-propandiol | - | 10 | - | - | - | 15 |
| Copolymer of dimethylterephthalate, 1,2-propylene glycol, methyl capped PEG | 2.0 | | | | | |
| Poly(ethyleneimine) ethoxylated, PEI600 E20 | | 1.8 | | | | |
| Acrylic acid/maleic acid copolymer | | | 2.9 | | | |
| Acusol 880 (Hydrophobically Modified Non-Ionic Polyol) | | | | 2.0 | 1.8 | 2.9 |
| Protease (55mg/g active) | - | - | - | - | 0.1 | 0.1 |
| Amylase (30mg/g active) | - | - | - | - | - | 0.02 |
| Brightener | 0.21 | - | - | 0.15 | - | 0.18 |
| Dye or mixture or dyes selected from Examples 1 - 28 in Table 1. | 0.01 | 0.00 5 | 0.00 6 | 0.00 2 | 0.00 7 | 0.00 8 |
| Additional Neat Perfume (2) | 0.5 | 0.5 | 0.5 | 0.5 | 0.5 | 0.5 |
| Amine (1) | 0.1 | 0.5 | 0.0 | 0.01 | 0.02 | 0.00 |
| Perfume Delivery System As Disclosed In The Present Specification Including Examples 2-3 | 0.05 | 0.0 | 0.1 | 0.0 | 0.2 | 0.4 |

(continued)

| Ingredients | 26 | 27 | 28 | 29 | 30 | 31 |
|---|---|---|---|---|---|---|
| Perfume comprising one or more PRMs from Table 1 | 0.3 | 0.4 | 0.01 | 0.02 | 0.04 | 0.1 |
| water, other optional agents/ components* | to 100 % bala nce | to 100 % bala nce | to 100 % bala nce | to 100 % bala nce | to 100 % bala nce | to 100 % bala nce |
| (3) One or more materials comprising an amine moiety as disclosed in the present specification.<br>(4) Optional.<br>*Other optional agents/components include suds suppressors, structuring agents such as those based on Hydrogenated Castor Oil (preferably Hydrogenated Castor Oil, Anionic Premix), solvents and/or Mica pearlescent aesthetic enhancer. | | | | | | |

Raw Materials and Notes For Composition Examples

LAS is linear alkylbenzenesulfonate having an average aliphatic carbon chain length $C_9$-$C_{15}$ supplied by Stepan, Northfield, Illinois, USA or Huntsman Corp. (HLAS is acid form).
$C_{12-14}$ Dimethylhydroxyethyl ammonium chloride, supplied by Clariant GmbH, Germany
AE3S is $C_{12-15}$ alkyl ethoxy (3) sulfate supplied by Stepan, Northfield, Illinois, USA
AE7 is $C_{12-15}$ alcohol ethoxylate, with an average degree of ethoxylation of 7, supplied by Huntsman, Salt Lake City, Utah, USA
AES is $C_{10-18}$ alkyl ethoxy sulfate supplied by Shell Chemicals.
AE9 is $C_{12-13}$ alcohol ethoxylate, with an average degree of ethoxylation of 9, supplied by Huntsman, Salt Lake City, Utah, USA
HSAS or HC1617HSAS is a mid-branched primary alkyl sulfate with average carbon chain length of about 16-17
Sodium tripolyphosphate is supplied by Rhodia, Paris, France
Zeolite A is supplied by Industrial Zeolite (UK) Ltd, Grays, Essex, UK
1.6R Silicate is supplied by Koma, Nestemica, Czech Republic
Sodium Carbonate is supplied by Solvay, Houston, Texas, USA
Polyacrylate MW 4500 is supplied by BASF, Ludwigshafen, Germany
Carboxymethyl cellulose is Finnfix® V supplied by CP Kelco, Arnhem, Netherlands Suitable chelants are, for example, diethylenetetraamine pentaacetic acid (DTPA) supplied by Dow Chemical, Midland, Michigan, USA or Hydroxyethane di phosphonate (HEDP) supplied by Solutia, St Louis, Missouri, USA Bagsvaerd, Denmark
Savinase®, Natalase®, Stainzyme®, Lipex®, Celluclean™, Mannaway® and Whitezyme® are all products of Novozymes, Bagsvaerd, Denmark.
Proteases may be supplied by Genencor International, Palo Alto, California, USA (e.g. Purafect Prime®) or by Novozymes, Bagsvaerd, Denmark (e.g. Liquanase®, Coronase®). Fluorescent Brightener 1 is Tinopal® AMS, Fluorescent Brightener 2 is Tinopal® CBS-X, Sulphonated zinc phthalocyanine and Direct Violet 9 is Pergasol® Violet BN-Z all supplied by Ciba Specialty Chemicals, Basel, Switzerland
Sodium percarbonate supplied by Solvay, Houston, Texas, USA
Sodium perborate is supplied by Degussa, Hanau, Germany
NOBS is sodium nonanoyloxybenzenesulfonate, supplied by Future Fuels, Batesville, USA
TAED is tetraacetylethylenediamine, supplied under the Peractive® brand name by Clariant GmbH, Sulzbach, Germany
S-ACMC is carboxymethylcellulose conjugated with C.I. Reactive Blue 19, sold by Megazyme, Wicklow, Ireland under the product name AZO-CM-CELLULOSE, product code S-ACMC.
Soil release agent is Repel-o-tex® PF, supplied by Rhodia, Paris, France
Acrylic Acid/Maleic Acid Copolymer is molecular weight 70,000 and acrylate:maleate ratio 70:30, supplied by BASF, Ludwigshafen, Germany
Na salt of Ethylenediamine-N,N'-disuccinic acid, (S,S) isomer (EDDS) is supplied by Octel, Ellesmere Port, UK
Hydroxyethane di phosphonate (HEDP) is supplied by Dow Chemical, Midland, Michigan, USA
Suds suppressor agglomerate is supplied by Dow Corning, Midland, Michigan, USA
HSAS is mid-branched alkyl sulfate as disclosed in US 6,020,303 and US 6,060,443
$C_{12-14}$ dimethyl Amine Oxide is supplied by Procter & Gamble Chemicals, Cincinnati, USA
Random graft copolymer is a polyvinyl acetate grafted polyethylene oxide copolymer having a polyethylene oxide backbone and multiple polyvinyl acetate side chains. The molecular weight of the polyethylene oxide backbone is about 6000 and the weight ratio of the polyethylene oxide to polyvinyl acetate is about 40:60 and no more than 1 grafting point per

50 ethylene oxide units.

Ethoxylated polyethyleneimine is polyethyleneimine (MW = 600) with 20 ethoxylate groups per -NH.

Cationic cellulose polymer is LK400, LR400 and/or JR30M from Amerchol Corporation, Edgewater NJ

Note: all enzyme levels are expressed as % enzyme raw material

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

Example 32: Shampoo Formulations

| Ingredient | |
|---|---|
| Ammonium Laureth Sulfate ($AE_3S$) | 6.00 |
| Ammonium Lauryl Sulfate (ALS) | 10.00 |
| Laureth-4 Alcohol | 0.90 |
| Trihydroxystearin (7) | 0.10 |
| Perfume comprising one or more PRMs from Table 1 | 0.60 |
| Sodium Chloride | 0.40 |
| Citric Acid | 0.04 |
| Sodium Citrate | 0.40 |
| Sodium Benzoate | 0.25 |
| Ethylene Diamine Tetra Acetic Acid | 0.10 |
| Dimethicone (9, 10, 11) | 1.00(9) |
| Water and Minors (QS to 100%) | Balance |

Example 33 - 35 : Fine Fragrance Formulations

| Ingredient | 33 | 34 | 35 |
|---|---|---|---|
| Cyclic oligosaccharide | 0 | 5 | 10 |
| Ethanol | 90 | 75 | 80 |
| Perfume comprising one or more PRMs from Table 1 | 10 | 20 | 10 |

Example 36- 49: Dentifrice Containing Sensate

| Ingredient | 36 | 37 | 38 | 39 | 40 | 41 | 42 | 43 | 44 |
|---|---|---|---|---|---|---|---|---|---|
| Calcium Peroxide | | | 0.1 | | | | | | |
| Carbomer 956 | 0.2 | | | 0.3 | 0.2 | 0.2 | 0.2 | 0.2 | 0.2 |
| CMC | | 0.75 | 0.2 | | | 1.0 | 1.0 | 1.0 | 1.0 |
| Color Solution (1%) | 0.05 | 0.05 | 0.5 | 0.75 | 0.18 | 0.02 | 0.25 | 0.05 | 0.05 |
| Wintergreen Spice Flavor | | | | | 0.15 | | | | |

(continued)

| Ingredient | 36 | 37 | 38 | 39 | 40 | 41 | 42 | 43 | 44 |
|---|---|---|---|---|---|---|---|---|---|
| Fruit Mint Flavor | | 0.55 | | | | | | | |
| Mint Flavor | 0.59 | | 0.45 | | 0.42 | 1.0 | 1.2 | 1.0 | 1.0 |
| Cinnamon Flavor | | | | 0.5 | | | | | |
| Vanillyl Butyl Ether | | | | | 0.02 | | | | |
| WS-23 | | | 0.1 | 0.05 | 0.1 | | | | |
| WS-3 | | | 0.2 | 0.05 | 0.2 | | | | |
| MGA | | | | 0.2 | | | | | |
| Menthol | 0.52 | 0.55 | 0.56 | 0.15 | 0.58 | | | | |
| Sensate comprising one or more PRMs from Table 1 | 0.01 | 0.03 | 0.015 | 0.004 | 0.01 | 0.01 | 0.03 | 0.008 | 0.02 |
| Potassium Sorbate | | | | | | 0.004 | 0.008 | 0.004 | 0.004 |
| Poloxamer 407 | | | 1.0 | | 0.2 | 0.2 | 0.2 | 0.2 | 0.2 |
| Polyethylene Glycol 300 | 3.0 | 3.0 | | 3.0 | | | | | |
| Polyethylene Glycol 600 | | | 2.3 | | | | | | |
| Propylene Glycol | | | 10.0 | | | | | | |
| Saccharin Sodium | 0.46 | 0.5 | 0.45 | 0.4 | 0.58 | 0.4 | 0.4 | 0.4 | 0.4 |
| Sucralose | | | | | | | 0.02 | 0.02 | 0.02 |
| Silica Abrasive | 22.0 | 31.0 | 20.0 | 21.0 | 17.0 | 15.0 | 15.0 | 15.0 | 15.0 |
| Sodium Benzoate | | | | | | 0.004 | 0.004 | 0.004 | 0.004 |
| Silica Thickening | | | 2.0 | | | 7.0 | 7.0 | 7.0 | 7.0 |
| Sodium Bicarbonate | | 1.5 | 9.0 | | | | | | |
| Sodium Carbonate | | 0.5 | | | | | | | |
| NaOH 50% soln | | | 1.74 | 2.2 | | 2.0 | 2.0 | 2.0 | 2.0 |
| Na Lauryl Sulfate (27.9% soln) | 4.0 | 5.0 | 3.0 | 4.0 | 4.0 | | | 3.0 | 2.0 |
| Stannous Fluoride | 0.454 | 0.454 | | | | | | | |
| Sodium Fluoride | | | | | | 0.243 | 0.243 | 0.243 | |
| Sodium MFP | | | 0.76 | 0.76 | 0.76 | 0.76 | | | |
| Glycerin USP 99.7% | 9.0 | 11.9 | 33.0 | 9.0 | | | | | |
| Sorbitol Soln USP | 24.3 | 24.5 | 4.0 | 44.7 | 56.9 | 43.0 | 43.0 | 40.0 | 38.0 |
| Tetra Na Pyrophosphate Anhydrous | 2.05 | 5.045 | 3.85 | | 3.85 | | | | |
| Tetra Potassium | 6.38 | | | | | | | | |
| Pyrophosphate (60%Soln) | | | | | | | | | |
| Na Acid Pyrophosphate | 2.1 | | | 4.0 | 1.0 | 4.3 | 4.5 | 4.5 | 2.0 |
| Alkyl Phosphate | | | | | | 3.5 | 6.7 | 3.5 | 3.5 |
| Cocamidopropyl Betaine (30%Soln) | | | | | | 3.5 | | | |
| Titanium Dioxide | 0.5 | | 1.0 | | 0.25 | 0.3 | 0.3 | 0.2 | 0.2 |
| Ti02/Carnauba Wax Prills | | 0.6 | | 0.3 | | | | | |

(continued)

| Ingredient | 36 | 37 | 38 | 39 | 40 | 41 | 42 | 43 | 44 |
|---|---|---|---|---|---|---|---|---|---|
| Xanthan Gum | 0.6 | | 0.4 | 0.45 | 0.7 | 0.3 | 0.3 | 0.3 | 0.3 |
| Water Purified USP | QS | QS | QS | QS | QS | QS | QS | QS | QS |
| | | | | | | | | | |
| Ingredient | 45 | 46 | 47 | 48 | 49 | | | | |
| Calcium Carbonate | | | | 40.0 | | | | | |
| Dibasic Calcium Phosphate Dihydrate | | | 35.0 | | | | | | |
| Silica Abrasive | 24.0 | 12.5 | | | 17.0 | | | | |
| Phytic Acid | | 0.8 | | | 2.0 | | | | |
| Gantrez S-97 | | | 2.0 | | | | | | |
| Color Solution (1%) | 0.05 | 0.05 | | | 0.05 | | | | |
| Saccharin sodium | | | | | 0.47 | 0.25 | 0.3 | 0.3 | 0.58 |
| Spice Mint Flavor | | | | | | | | 1.0 | |
| Wintergreen Spice Flavor | | | | | | 1.2 | | | 0.15 |
| Mint Flavor | | | | | | 0.3 | 0.6 | 0.5 | 0.42 |
| Cinnamon Flavor | | | | | 0.18 | | | | |
| WS-23 Coolant | | | | | 0.03 | | | | 0.02 |
| WS-3 Coolant | | | | | 0.03 | | | | 0.02 |
| MGA | | | | | 0.08 | 0.08 | | | |
| Menthol | | | | | 0.38 | 0.24 | 0.2 | 0.5 | 0.58 |
| Sensate comprising one or more PRMs from Table 1 | | | | | 0.08 | 0.005 | 0.004 | 0.008 | 0.01 |
| Glycerin | | | | | 16.5 | | 15.0 | | |
| Sorbitol solution | | | | | 10.5 | 33.0 | 11.5 | 14.0 | 57.0 |
| Poloxamer 407 | | | | | | | | | 0.2 |
| Polyethylene Glycol 300 | | | | | | | | 2.5 | |
| Polyethylene Glycol 600 | | | | | | | 3.0 | | |
| Carbomer | | | | | | 0.3 | | | 0.2 |
| CMC 7M8SF | | | | | 1.0 | 1.0 | 1.0 | 1.0 | |
| HEC 250MX | | | | | | 0.5 | | | |
| Sodium Lauryl Sulfate (27.9% soln) | | | | | 7.5 | 7.0 | 5.5 | 7.0 | 4.0 |
| NaOH 50% soln | | | | | | 1.0 | | | |
| Sodium Monofluorophosphate | | | | | 0.76 | | 0.76 | 0.76 | 0.76 |
| Sodium Fluoride | | | | | | 0.32 | | | |
| Sodium Gluconate | | | | | | 1.0 | | | |
| Stannous Chloride Dihydrate | | | | | | 1.0 | | | |
| Zinc Citrate | | | | | | 0.5 | | | |
| Potassium Nitrate | | | | | 5.0 | | | | |
| Sodium Phosphate, Tribasic | | | | | 3.2 | | | | |

(continued)

| Ingredient | 36 | 37 | 38 | 39 | 40 | 41 | 42 | 43 | 44 |
|---|---|---|---|---|---|---|---|---|---|
| Tetra Sodium Pyrophosphate, Anhydrous | | | | | | | 0.5 | 0.5 | 3.85 |
| Sodium Acid Pyrophosphate | | | | | | | | | 1.0 |
| Titanium Dioxide | | | | | 0.5 | 0.5 | | | 0.25 |
| Xanthan Gum (Keltro 1000 | | | | | | | | 0.5 | 0.7 |
| Carrageenan | | | | | | 0.5 | | | |
| Water, Purified, USP | | | | | QS | QS | QS | QS | QS |

Example 50-52: Mouthrinse Containing Sensate

| Ingredient | 50 | 51 | 52 |
|---|---|---|---|
| Ethanol USP 190 proof | 15 | 15 | 15 |
| Glycerin | 7.5 | 7.5 | 7.5 |
| Polysorbate 80 NF | 0.12 | 0.12 | 0.12 |
| Flavor' | 0.16 | 0.16 | 0.16 |
| Saccharin sodium | 0.067 | 0.067 | 0.06 |
| Color solution | 0.04 | 0.04 | 0.04 |
| Sensate comprising one or more PRMs from Table 1 | 0.03 | 0.017 | 1 |
| Calcium chloride | 0.025 | 0.025 | 0.025 |
| Cetylpyridinium chloride | 0.045 | 0.045 | 0.045 |
| Benzoic acid | 0.005 | 0.005 | 0.005 |
| Sodium benzoate | 0.054 | 0.054 | 0.054 |
| Water | QS | QS | QS |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

The citation of any document of the Detailed Description is not to be construed as an admission that it is prior art with respect to the present invention. To the extent that any meaning or definition of a term in this document conflicts with any meaning or definition of the same term in a citation the meaning or definition assigned to that term in this document shall govern.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A molecule selected from the group consisting of 5-(but-2-enoyl)-4,4,6-trimethylcyclohex-2-en-1-yl acetate.

2. A molecule selected from the group consisting of 3,4-dimethyl-1-(*R,S*)-(4-methylpent-3-en-1-yl)cyclohex-3-enecarbonitrile and stereoisomers thereof.

3. A molecule selected from the group consisting of (*R,S*)-(3,4-dimethylcyclohex-3-enyl)methyl acetate and stereoisomers thereof.

4. A molecule selected from the group consisting of 1-(3-ethoxy-4-hydroxyphenyl)pentan-1-one and 1-(3-ethoxy-4-hydroxyphenyl)-3-methylbutan-1-one.

5. A perfume or sensate composition comprising, based on total perfume weight, from 0.01% to 50%, preferably from 0.1% to 15%, more preferably from 0.1% to 10%, most preferably from 0.5% to 10% of one or more molecules according to any one of claims 2 or 4; and mixtures thereof; and an optional solvent.

6. A consumer product comprising, based on total consumer product weight, from 0.0001% to 25%, preferably from 0.0005% to 10%, more preferably from 0.001% to 5%, more preferably from 0.005% to 2.5%, most preferably from 0.01% to 1% of one or more molecules according to any one of claims 1 to 4; and mixtures thereof; and an adjunct ingredient.

7. A consumer product according to Claim 6, said consumer product being a cleaning and/or treatment composition, said composition comprising, based on total composition weight, from 0.0001% to 25%, preferably from 0.0005% to 10%, more preferably from 0.001% to 5%, more preferably from 0.005% to 2.5%, most preferably from 0.01% to 1% of one or more molecules according to any one of claims 1 to 4; and mixtures thereof.

8. A consumer product according to any one of Claims 6 to 7, said consumer product being a fabric and/or hard surface cleaning and/or treatment composition, said composition comprising, based on total composition weight, from 0.00001% to 25%, preferably from 0.00005% to 10%, more preferably from 0.0001% to 5%, more preferably from 0.0005% to 1.0%, most preferably from 0.001% to 0.5% of one or more molecules according to any one of claims 1 to 4 and mixtures thereof.

9. A consumer product according to Claim 6, said consumer product being a detergent, said detergent comprising, based on total detergent weight, from 0.00001% to 25%, preferably from 0.00005% to 10%, more preferably from 0.0001% to 5%, more preferably from 0.0005% to 1.0%, most preferably from 0.001% to 0.5% of one or more molecules according to any one of claims 1 to 4 and mixtures thereof.

10. A consumer product according to Claim 6, said consumer product being a highly compacted consumer product, preferably a highly compacted detergent, said highly compacted consumer product comprising, based on total highly compacted consumer product weight, from 0.00001% to 25%, preferably from 0.00005% to 10%, more preferably from 0.0001% to 5%, more preferably from 0.0005% to 1.0%, most preferably from 0.001% to 0.5% of one or more molecules according to any one of claims 1 to 4 and mixtures thereof.

11. A perfume delivery system comprising from 0.001% to 50%, preferably from 0.005% to 30%, more preferably from 0.01% to 10%, more preferably from 0.025% to 5%, most preferably from 0.025% to 1% of one or more molecules according to any one of claims 1 to 4 and mixtures thereof; wherein said perfume delivery system is selected from a polymer assisted delivery system; a molecule-assisted delivery system; a fiber-assisted delivery system; an amine assisted delivery system; a cyclodextrin delivery system; a starch encapsulated accord; and/or an inorganic carrier delivery system; or a pro-perfume;

(i) preferably when said perfume delivery system is a nanocapsule or a microcapsule, said perfume delivery system comprises, based on total nanocapsule or microcapsule weight, from 0.1% to 99%, preferably from 25% to 95%, more preferably from 30 to 90%, more preferably from 45% to 90%, most preferably from 65% to 90% of one or more molecules according to any one of claims 1 to 4; and mixtures thereof;
(ii) preferably when said perfume delivery system is a starch encapsulated accord, said perfume delivery system comprises, based on total starch encapsulate or starch agglomerate weight, from 0.1% to 99%, preferably from 25% to 95%, more preferably from 30 to 90%, more preferably from 45% to 90%, most preferably from 65% to 90% of one or more molecules according to any one of claims 1 to 4; and mixtures thereof;
(iii) preferably when said perfume delivery system is a cyclodextrin delivery system, said perfume delivery system comprises, based on total cyclodextrin delivery system weight, from 0.1% to 99%, preferably from 2.5% to 75%, more preferably from 5% to 60%, more preferably from 5% to 50%, most preferably from 5% to 25% of one or more molecules according to any one of claims 1 to 4 and mixtures thereof;
(iv) preferably when said perfume delivery system is a polymer assisted delivery matrix system, said perfume delivery system comprises, based on total polymer assisted delivery matrix system weight, from 0.1% to 99%,

preferably from 2.5% to 75%, more preferably from 5% to 60%, more preferably from 5% to 50%, most preferably from 5% to 25% of one or more molecules according to any one of claims 1 to 4 and mixtures thereof;
(v) preferably when said perfume delivery system is an amine assisted delivery system , said perfume delivery system comprises, based on total amine assisted delivery system weight, from 1% to 99%, preferably from 2.5% to 75%, more preferably from 5% to 60%, more preferably from 5% to 50%, most preferably from 5% to 25% of one or more molecules according to any one of claims 1 to 4; and mixtures thereof;
(vi) preferably when said perfume delivery system is a pro-perfume amine reaction product, said perfume delivery system comprises, based on total pro-perfume amine reaction product weight, from 0.1% to 99%, preferably from 1% to 99%, more preferably from 5% to 90%, more preferably from 10% to 75%, more preferably from 20% to 75%, most preferably from 25% to 60% of one or more molecules according to claim 4; and mixtures thereof.

**12.** A consumer product comprising, based on total consumer product weight, from 0.001% to 20%, preferably from 0.01% to 10%, more preferably from 0.05% to 5%, most preferably from 0.1% to 0.5% of a perfume delivery system selected from the perfume delivery systems of claim 11 and mixtures thereof.

**13.** A method of counteracting a malodor comprising contacting a situs with one or more molecules selected from the group consisting of molecules according to claims 2 or 4 and mixtures thereof, preferably, said situs comprises said malodor, more preferably, said malodor comprises a thiol moiety.

**Patentansprüche**

**1.** Molekül, ausgewählt aus der Gruppe bestehend aus 5-(But-2-enoyl)-4,4,6-trimethylcyclohex-2-en-1-ylacetat.

**2.** Molekül, ausgewählt aus der Gruppe bestehend aus 3,4-Dimethyl-1-(*R,S*)-(4-methylpent-3-en-1-yl)cyclohex-3-encarbonitril und Stereoisomeren davon.

**3.** Molekül, ausgewählt aus der Gruppe bestehend aus (R,S)-(3,4-Dimethylcyclohex-3-enyl) methylacetat und Stereoisomeren davon.

**4.** Molekül, ausgewählt aus der Gruppe bestehend aus 1-(3-Ethoxy-4-hydroxyphenyl) pentan-1-on und 1-(3-Ethoxy-4-hydroxyphenyl)-3-methylbutan-1-on.

**5.** Duftstoff oder sinnlich wahrgenommene Zusammensetzung, umfassend, bezogen auf das Gesamtgewicht des Duftstoffes, von 0,01 % bis 50 %, vorzugsweise von 0,1 % bis 15 %, mehr bevorzugt von 0,1 % bis 10 %, am meisten bevorzugt von 0,5 % bis 10 % ein oder mehrere Moleküle nach einem der Ansprüche 2 oder 4; und Mischungen davon; und ein optionales Lösungsmittel.

**6.** Endprodukt, umfassend, bezogen auf das Gesamtgewicht des Endprodukts, von 0,0001 % bis 25 %, vorzugsweise von 0,0005 % bis 10 %, mehr bevorzugt von 0,001 % bis 5 %, mehr bevorzugt von 0,005 % bis 2,5 %, am meisten bevorzugt von 0,01 % bis 1 % ein oder mehrere Moleküle nach einem der Ansprüche 1 bis 4; und Mischungen davon; und einen Zusatzbestandteil.

**7.** Endprodukt nach Anspruch 6, wobei das Endprodukt eine Reinigungs- und/oder Behandlungszusammensetzung ist, wobei die Zusammensetzung, bezogen auf das Gesamtgewicht der Zusammensetzung, von 0,0001 % bis 25 %, vorzugsweise von 0,0005 % bis 10 %, mehr bevorzugt von 0,001 % bis 5 %, mehr bevorzugt von 0,005 % bis 2,5 %, am meisten bevorzugt von 0,01 % bis 1 % ein oder mehrere Moleküle nach einem der Ansprüche 1 bis 4 umfasst; und Mischungen davon umfasst.

**8.** Endprodukt nach einem der Ansprüche 6 bis 7, wobei das Endprodukt eine Reinigungs- und/oder Behandlungszusammensetzung für Stoff und/oder harte Oberflächen ist, wobei die Zusammensetzung, bezogen auf das Gesamtgewicht der Zusammensetzung, von 0,00001 % bis 25 %, vorzugsweise von 0,00005 % bis 10 %, mehr bevorzugt von 0,0001 % bis 5 %, mehr bevorzugt von 0,0005 % bis 1,0 %, am meisten bevorzugt von 0,001 % bis 0,5 % ein oder mehrere Moleküle nach einem der Ansprüche 1 bis 4 und Mischungen davon umfasst.

**9.** Endprodukt nach Anspruch 6, wobei das Endprodukt ein Waschmittel ist, wobei das Waschmittel, bezogen auf das Gesamtgewicht des Waschmittels, von 0,00001 % bis 25 %, vorzugsweise von 0,00005 % bis 10 %, mehr bevorzugt

von 0,0001 % bis 5 %, mehr bevorzugt von 0,0005 % bis 1,0 %, am meisten bevorzugt von 0,001 % bis 0,5 % ein oder mehrere Moleküle nach einem der Ansprüche 1 bis 4 und Mischungen davon umfasst.

**10.** Endprodukt nach Anspruch 6, wobei das Endprodukt ein stark verdichtetes Endprodukt, vorzugsweise ein stark verdichtetes Waschmittel ist, wobei das stark verdichtete Endprodukt, bezogen auf das Gesamtgewicht des stark verdichteten Endprodukts, von 0,00001 % bis 25 %, vorzugsweise von 0,00005 % bis 10 %, mehr bevorzugt von 0,0001 % bis 5 %, mehr bevorzugt von 0,0005 % bis 1,0 %, am meisten bevorzugt von 0,001 % bis 0,5 % ein oder mehrere Moleküle nach einem der Ansprüche 1 bis 4 und Mischungen davon umfasst.

**11.** Duftstoffabgabesystem, umfassend von 0,001 % bis 50 %, vorzugsweise von 0,005 % bis 30 %, mehr bevorzugt von 0,01 % bis 10 %, mehr bevorzugt von 0,025 % bis 5 %, am meisten bevorzugt von 0,025 % bis 1 % ein oder mehrere Moleküle nach einem der Ansprüche 1 bis 4 und Mischungen davon; wobei das Duftstoffabgabesystem ausgewählt ist aus einem polymerunterstützten Abgabesystem; einem molekülunterstützten Abgabesystem; einem faserunterstützten Abgabesystem; einem aminunterstützten Abgabesystem; einem Cyclodextrinabgabesystem; einer in Stärke eingekapselten Duftnote; und/oder einem anorganischen Trägerabgabesystem; oder einem Pro-Duftstoff;

(i) wenn das Duftstoffabgabesystem vorzugsweise eine Nanokapsel oder eine Mikrokapsel ist, umfasst das Duftstoffabgabesystem, bezogen auf das Gesamtgewicht der Nanokapsel- oder Mikrokapsel, von 0,1 % bis 99 %, vorzugsweise von 25 % bis 95 %, mehr bevorzugt von 30 bis 90 %, mehr bevorzugt von 45 % bis 90 %, am meisten bevorzugt von 65 % bis 90 %, ein oder mehrere Moleküle nach einem der Ansprüche 1 bis 4; und Mischungen davon;
(ii) wenn das Duftstoffabgabesystem vorzugsweise eine mit Stärke eingekapselte Duftnote ist, umfasst das Duftstoffabgabesystem, bezogen auf das Gesamtgewicht der eingekapselten Gesamtstärke oder dem Stärke-Agglomerat, von 0,1 % bis 99 %, vorzugsweise von 25 % bis 95 %, mehr bevorzugt von 30 bis 90 %, mehr bevorzugt von 45 % bis 90 %, am meisten bevorzugt von 65 % bis 90 % ein oder mehrere Moleküle nach einem der Ansprüche 1 bis 4; und Mischungen davon;
(iii) wenn das Duftstoffabgabesystem vorzugsweise ein Cyclodextrinabgabesystem ist, umfasst das Duftstoffabgabesystem, bezogen auf das Gesamtgewicht des Cyclodextrinabgabesystems, von 0,1 % bis 99 %, vorzugsweise von 2,5 % bis 75 %, mehr bevorzugt von 5 % bis 60 %, mehr bevorzugt von 5 % bis 50 %, besonders bevorzugt von 5 % bis 25 % ein oder mehrere Moleküle nach einem der Ansprüche 1 bis 4 und Mischungen davon;
(iv) wenn das Duftstoffabgabesystem vorzugsweise ein polymerunterstütztes Abgabematrixsystem ist, umfasst das Duftstoffabgabesystem, bezogen auf das Gesamtgewicht des polymerunterstützten Abgabematrixsystems, von 0,1 % bis 99 %, vorzugsweise von 2,5 % bis 75 %, mehr bevorzugt von 5 % bis 60 %, mehr bevorzugt von 5 % bis 50 %, am meisten bevorzugt von 5 % bis 25 % eines oder mehrerer Moleküle nach einem der Ansprüche 1 bis 4 und Mischungen davon;
(v) wenn das Duftstoffabgabesystem vorzugsweise ein aminunterstütztes Abgabesystem ist, umfasst das Duftstoffabgabesystem, bezogen auf das Gesamtgewicht des aminunterstützten Abgabesystems, von 1 % bis 99 %, vorzugsweise von 2,5 % bis 75 %, mehr bevorzugt von 5 % bis 60 %, mehr bevorzugt von 5 % bis 50 %, am meisten bevorzugt von 5 % bis 25 % ein oder mehrere Moleküle nach einem der Ansprüche 1 bis 4; und Mischungen davon;
(vi) wenn das Duftstoffabgabesystem vorzugsweise ein Pro-Duftstoffamin-Reaktionsprodukt ist, umfasst das Duftstoffabgabesystem, bezogen auf das Gesamtgewicht des Pro-Duftstoffamin-Reaktionsprodukts, von 0,1 % bis 99 %, vorzugsweise von 1 % bis 99 %, mehr bevorzugt von 5 % bis 90 %, mehr bevorzugt von 10 % bis 75 %, mehr bevorzugt von 20 % bis 75 %, am meisten bevorzugt von 25 % bis 60 %, ein oder mehrere Moleküle nach Anspruch 4; und Mischungen davon.

**12.** Endprodukt umfassend, bezogen auf das Gesamtgewicht des Endprodukts, von 0,001 % bis 20 %, vorzugsweise von 0,01 % bis 10 %, mehr bevorzugt von 0,05 % bis 5 %, am meisten bevorzugt von 0,1 % bis 0,5 % ein Dufstoffabgabesystem, ausgewählt aus den Duftstoffabgabesystemen nach Anspruch 11 und Mischungen davon.

**13.** Verfahren zur Bekämpfung eines schlechten Geruchs, umfassend das Inkontaktbringen einer Stelle mit einem oder mehreren Molekülen, ausgewählt aus der Gruppe bestehend aus Molekülen nach Anspruch 2 oder 4 und Mischungen davon, wobei die Stelle den schlechten Geruch umfasst, wobei der schlechte Geruch mehr bevorzugt eine Thioleinheit umfasst.

## Revendications

1. Molécule choisie dans le groupe constitué d'acétate de 5-(but-2-énoyl)-4,4,6-trimethylcyclohex-2-én-1-yle.

2. Molécule choisie dans le groupe constitué de 3,4-diméthyl-1-(*R,S*)-(4-méthylpent-3-én-1-yl)cyclohex-3-ènecarbonitrile et des stéréo-isomères de celui-ci.

3. Molécule choisie dans le groupe constitué d'acétate de (*R,S*)-(3,4-diméthylcyclohex-3-ényl)méthyle et des stéréo-isomères de celui-ci.

4. Molécule choisie dans le groupe constitué de 1-(3-éthoxy-4-hydroxyphényl)pentan-1-one et 1-(3-éthoxy-4-hydroxyphényl)-3-méthylbutan-1-one.

5. Composition de parfum ou d'agent sensoriel comprenant, sur la base du poids total de parfum, de 0,01 % à 50 %, de préférence de 0,1 % à 15 %, plus préférablement de 0,1 % à 10 %, le plus préférablement de 0,5 % à 10 % d'une ou plusieurs molécules selon l'une quelconque des revendications 2 ou 4 ; et leurs mélanges ; et un solvant facultatif.

6. Produit de consommation comprenant, sur la base du poids total de produit de consommation, de 0,0001 % à 25 %, de préférence de 0,0005 % à 10 %, plus préférablement de 0,001 % à 5 %, plus préférablement de 0,005 % à 2,5 %, le plus préférablement de 0,01 % à 1 % d'une ou plusieurs molécules selon l'une quelconque des revendications 1 à 4 ; et leurs mélanges ; et un ingrédient additif.

7. Produit de consommation selon la revendication 6, ledit produit de consommation étant une composition de nettoyage et/ou de traitement, ladite composition comprenant, sur la base du poids total de la composition, de 0,0001 % à 25 %, de préférence de 0,0005 % à 10 %, plus préférablement de 0,001 % à 5 %, plus préférablement de 0,005 % à 2,5 %, le plus préférablement de 0,01 % à 1 % d'une ou plusieurs molécules selon l'une quelconque des revendications 1 à 4 ; et leurs mélanges.

8. Produit de consommation selon l'une quelconque des revendications 6 ou 7, ledit produit de consommation étant une composition de nettoyage et/ou de traitement des tissus et/ou des surfaces dures, ladite composition comprenant, sur la base du poids total de la composition, de 0,00001 % à 25 %, de préférence de 0,00005 % à 10 %, plus préférablement de 0,0001 % à 5 %, plus préférablement de 0,0005 % à 1,0 %, le plus préférablement de 0,001 % à 0,5 % d'une ou plusieurs molécules selon l'une quelconque des revendications 1 à 4 et leurs mélanges.

9. Produit de consommation selon la revendication 6, ledit produit de consommation étant un détergent, ledit détergent comprenant, sur la base du poids total du détergent, de 0,00001 % à 25 %, de préférence de 0,00005 % à 10 %, plus préférablement de 0,0001 % à 5 %, plus préférablement de 0,0005 % à 1,0 %, le plus préférablement de 0,001 % à 0,5 % d'une ou plusieurs molécules selon l'une quelconque des revendications 1 à 4 et leurs mélanges.

10. Produit de consommation selon la revendication 6, ledit produit de consommation étant un produit de consommation fortement compacté, de préférence un détergent fortement compacté, ledit produit de consommation fortement compacté comprenant, sur la base du poids total de produit de consommation fortement compacté, de 0,00001 % à 25 %, de préférence de 0,00005 % à 10 %, plus préférablement de 0,0001 % à 5 %, plus préférablement de 0,0005 % à 1,0 %, le plus préférablement de 0,001 % à 0,5 % d'une ou plusieurs molécules selon l'une quelconque des revendications 1 à 4 et leurs mélanges.

11. Système de libération de parfum comprenant de 0,001 % à 50 %, de préférence de 0,005 % à 30 %, plus préférablement de 0,01 % à 10 %, plus préférablement de 0,025 % à 5 %, le plus préférablement de 0,025 % à 1 % d'une ou plusieurs molécules selon l'une quelconque des revendications 1 à 4 et leurs mélanges ; dans lequel ledit système de libération de parfum est choisi parmi un système de libération assistée par polymère ; un système de libération assistée par molécule ; un système de libération assistée par fibre ; un système de libération assistée par une aminé ; un système de libération par cyclodextrine ; un accord encapsulé dans de l'amidon ; et/ou un système de libération par véhicule inorganique ; ou un proparfum ;

    (i) de préférence, lorsque ledit système de libération de parfum est une nanogélule ou une microgélule, ledit système de libération de parfum comprend, sur la base du poids total de nanogélule ou de microgélule, de 0,1 % à 99 %, de préférence de 25 % à 95 %, plus préférablement de 30 à 90 %, plus préférablement de 45 % à

90 %, le plus préférablement de 65 % à 90 % d'une ou plusieurs molécules selon l'une quelconque des revendications 1 à 4 ; et leurs mélanges ;

(ii) de préférence, lorsque ledit système de libération de parfum est un accord encapsulé dans de l'amidon, ledit système de libération de parfum comprend, sur la base du poids total d'encapsulation d'amidon ou d'agglomérat d'amidon, de 0,1 % à 99 %, de préférence de 25 % à 95 %, plus préférablement de 30 à 90 %, plus préférablement de 45 % à 90 %, le plus préférablement de 65 % à 90 % d'une ou plusieurs molécules selon l'une quelconque des revendications 1 à 4 ; et leurs mélanges ;

(iii) de préférence, lorsque ledit système de libération de parfum est un système de libération par cyclodextrine, ledit système de libération de parfum comprend, sur la base du poids total de système de libération par cyclodextrine, de 0,1 % à 99 %, de préférence de 2,5 % à 75 %, plus préférablement de 5 % à 60 %, plus préférablement de 5 % à 50 %, le plus préférablement de 5 % à 25 % d'une ou plusieurs molécules selon l'une quelconque des revendications 1 à 4 et leurs mélanges ;

(iv) de préférence, lorsque ledit système de libération de parfum est un système de matrice de libération assistée par polymère, ledit système de libération de parfum comprend, sur la base du poids total de système de matrice de libération assistée par polymère, de 0,1 % à 99 %, de préférence de 2,5 % à 75 %, plus préférablement de 5 % à 60 %, plus préférablement de 5 % à 50 %, le plus préférablement de 5 % à 25 % d'une ou plusieurs molécules selon l'une quelconque des revendications 1 à 4 et leurs mélanges ;

(v) de préférence, lorsque ledit système de libération de parfum est un système de libération assistée par une amine, ledit système de libération de parfum comprend, sur la base du poids total de système de libération assistée par une amine, de 1 % à 99 %, de préférence de 2,5 % à 75 %, plus préférablement de 5 % à 60 %, plus préférablement de 5 % à 50 %, le plus préférablement de 5 % à 25 % d'une ou plusieurs molécules selon l'une quelconque des revendications 1 à 4 ; et leurs mélanges ;

(vi) de préférence, lorsque ledit système de libération de parfum est un produit de réaction d'amine à proparfum, ledit système de libération de parfum comprend, sur la base du poids total de produit de réaction d'amine à proparfum, de 0,1 % à 99 %, de préférence de 1 % à 99 %, plus préférablement de 5 % à 90 %, plus préférablement de 10 % à 75 %, plus préférablement de 20 % à 75 %, le plus préférablement de 25 % à 60 % d'une ou plusieurs molécules selon la revendication 4 ; et leurs mélanges.

**12.** Produit de consommation comprenant, sur la base du poids total de produit de consommation, de 0,001 % à 20 %, de préférence de 0,01 % à 10 %, plus préférablement de 0,05 % à 5 %, le plus préférablement de 0,1 % à 0,5 % d'un système de libération de parfum choisi parmi les systèmes de libération de parfum selon la revendication 11 et leurs mélanges.

**13.** Procédé pour contrecarrer une mauvaise odeur comprenant la mise en contact d'un site avec une ou plusieurs molécules choisies dans le groupe constitué de molécules selon les revendications 2 ou 4 et leurs mélanges, de préférence, ledit site comprend ladite mauvaise odeur, plus préférablement, ladite mauvaise odeur comprend un fragment thiol.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20070275866 A1
- US 20080305977 A1
- US 20040110648 A1
- US 7119060 B
- US 20050003980 A1
- US P6458754 B1
- US 5651976 A
- US 20060020459 A1
- US 7018978 B
- US 6326348 B1
- US 4430243 A
- US 5576282 A
- US 5597936 A
- US 6225464 B
- US 6020303 A
- US 6060443 A


### Non-patent literature cited in the description


- **A. LEO.** Comprehensive Medicinal Chemistry. Pergamon Press, 1990, vol. 4, 295